# EUROPEAN PATENT APPLICATION

(11) **EP 2 486 799 A1**
(43) Date of publication of application: **15.08.2012**
(21) Application number: 11154413.6
(22) Date of filing: 14.02.2011
(51) Int. Cl.: A21D 8/04, C12N 9/28

(54) **Method to produce cake with lipolytic enzyme and alpha-amylase**

(71) Applicant: DSM IP Assets B.V., 6411 TE Heerlen (NL)
(72) Inventor: Mastenbroek, José, 2496 HL Den Haag (NL); Carr, Neil, Wirral, CH47 6BG (GB); Stolze-Lagerweij, Helma Arina, 2678 WN De Lier (NL)
(74) Representative: Kranenburg-van Dijk, Saskia

(57) **Abstract**

The present invention describes a method to produce cake wherein in the preparation of the cake batter a baking composition comprising a lipolytic enzyme and an anti staling amylase or a cake pre-mix comprising flour and a baking composition comprising a lipolytic enzyme and an anti staling amylase is added in an effective amount, wherein the lipolytic enzyme is an isolated polypeptide having the amino acid sequence according to the mature polypeptide sequence in SEQ ID NO: 2 or an isolated polypeptide being at least 70% identical to said mature polypeptide sequence, and wherein the anti staling amylase is an isolated polypeptide according to SEQ ID NO: 9 or an isolated polypeptide being at least 70% identical thereto.

## Description

### Field of the invention

The present invention relates to a method to produce cake wherein a baking composition comprising a lipolytic enzyme and an anti staling amylase is used. The present invention also relates to the use of said baking composition in the production of a cake to improve cake or batter properties and/or to allow reduction of fat and/or eggs in the cake. The invention further relates to the baking compositions and to cake pre-mixes comprising the same.

### Background of the invention

Cakes are sweet baked products conventionally made of at least three main ingredients being eggs, sugar and flour and exist in a broad variety of formulations. Optionally, baking powder, water, fat - such as for example butter, margarine and or oil and other ingredients are added.

Cakes are made starting from a cake batter, i.e. a viscous, semi-liquid preparation made by beating or whipping the cake ingredients together and incorporating air. Commonly addition of leavening agents in the form of baking powder, expands the air bubbles present in the batter. Additionally the baking process itself leads to an expansion of the entrained gas bubbles; ultimately the liquid batter transforms into a solid cake-structure as the starch of the flour gelatinizes and the protein of the egg denatures; cakes most usually then have a fluffy, fine grained, sponge like and tender texture, rich in aroma and sweet in taste.

Cakes can be divided in two main categories. The shortening-based types of cakes (such as for example pound cakes), have a structure which is derived from a fat-liquid emulsion that is obtained during batter processing. The foam-based types of cakes (such as sponge cakes), which often do not comprise fat and wherein the structure is mainly derived from the foaming properties of eggs or other surfactants.

The type of cake ingredients and the ratio between them are important in determining cake properties such as e.g. crumb structure and cake volume. Each cake ingredient has one or more characteristics which result in particular cake properties. Flour is involved in the formation of the cake structure and in particular of the crumb structure of most cake types. Sugar is a flavouring ingredient which is also responsible for softening effect on the crumb and for retaining of moisture in the cake.

Eggs have multiple functionalities, depending also on the type of egg product which is used in the cake production. Egg whites are rich in moisture and therefore function as moisturiser and because of the presence of albumen protein as structure builder. Egg yolks are less rich in moisture, are rich in lipids and protein and therefore also function as structure builder, tenderiser and moisturiser. The function of whole eggs is similar to those of egg yolks. Egg powders are mainly structure formers. Shortening is responsible for favouring incorporation of air in the cake batter. Furthermore shortening tenderizes the cake and improves the cake shelf life. Leavening imparts volume and fluffiness to the final cake.

A balanced cake formula is one in which the ingredients are present in such ratios as to yield a good quality cake. Changing of the balance between the various ingredients (e.g. when aiming at producing cake with reduced level of egg, fat or sugar) requires compensating the changes by using the other formula components or by using baking additives such as enzymes and/or other baking ingredients.

WO2008092907 e.g. describes the use of phospholipase A in the production of cake to improve one or more cake properties and to allow the reduction of eggs and/or fats in the cake recipe.

The type of ingredients used to produce the cake and the specific cake recipe, as well as other external factors, may influence the shelf life of the final cake product.

The main factors that influence cake shelf life are mould growth, moisture migration and staling. Cake staling results in an increase of hardness of the crumb and the decrease of elasticity of the crumb. The short shelf life of some type of cakes especially due to the staling thereof is undesirable because cake producers are shifting from an artisanal, local-based production and sales to an industrialised and global manufacturing and sales. Therefore it is necessary that cake characteristics such as e.g. perceived freshness, moistness and softness of the cake crumb are maintained for weeks, months or, depending on the type of cake, even as long as a year after the date of cake manufacture.

The problem related to the staling of baked products is addressed in the prior art.

WO91/04669 discloses a process for retarding the staling of a baked product using a maltogenic α-amylase, e.g. one encoded by a DNA sequence derived from *Bacillus* strain NCIB11837.

WO99/43793 describes among others maltogenic α-amylase variants capable of forming cyclodextrins when acting on starch, while WO99/43794 describes maltogenic α-amylase variants with improved properties.

WO99/53769 discloses a baking composition comprising a phospholipase and an anti staling amylase and its use in the production of baked products.

WO2006/032281 and WO2008/148845 describe maltogenic α-amylase variants with high tolerance for sucrose which are particularly suitable in the production of cakes.

There is a need for alternative means of reducing staling in cake products and/or to improve overall cake properties in regular and eggs and/or fat reduced cakes.

It is an object of the present invention to provide alternative ways of reducing the staling of cake products and of improving cake properties.

### Summary of the invention

The present invention relates to a method to produce a cake comprising:
a) preparing a cake batter starting from cake batter ingredients,
b) baking the cake batter to yield the cake,
wherein in the preparation of the cake batter a baking composition comprising a lipolytic enzyme and an anti staling amylase or a cake pre-mix comprising flour and a baking composition comprising a lipolytic enzyme and an anti staling amylase is added in an effective amount,
wherein the lipolytic enzyme is an isolated polypeptide having the amino acid sequence according to the mature polypeptide sequence in SEQ ID NO: 2 or an isolated polypeptide being at least 70% identical to said mature polypeptide sequence, wherein the anti staling amylase is preferably an isolated polypeptide according to SEQ ID NO: 9 or an isolated polypeptide being at least 70% identical thereto.

The present invention also relates to the use of a baking composition comprising a lipolytic enzyme and an anti staling amylase or of a cake pre-mix comprising flour and a baking composition comprising a lipolytic enzyme and an anti staling amylase in the production of cake to improve one or more batter or cake properties, and/or to allow the reduction of eggs and/or fat in the cake, wherein the lipolytic enzyme is an isolated polypeptide having the amino acid sequence according to the mature polypeptide sequence in SEQ ID NO: 2 or an isolated polypeptide being at least 70% identical to said mature polypeptide sequence and wherein the anti staling amylase is preferably an isolated polypeptide according to SEQ ID NO: 9 or an isolated polypeptide being at least 70% identical thereto.

The present invention also relates to a baking compositions and cake pre-mixes comprising a baking composition comprising a lipolytic enzyme and an anti staling amylase, wherein the lipolytic enzyme is an isolated polypeptide having the amino acid sequence according to the mature polypeptide sequence in SEQ ID NO: 2 or an isolated polypeptide being at least 70% identical to said mature polypeptide sequence, wherein the anti staling amylase is preferably an isolated polypeptide according to SEQ ID NO: 9 or an isolated polypeptide being at least 70% identical thereto.

### Detailed description of the invention

In a first aspect the invention provides a method to produce a cake comprising:
a) preparing a cake batter starting from cake batter ingredients,
b) baking the cake batter to yield the cake,
wherein in the preparation of the cake batter a baking composition comprising a lipolytic enzyme and an anti staling amylase or a cake pre-mix comprising flour and a baking composition comprising a lipolytic enzyme and an anti staling amylase is added in an effective amount,
wherein the lipolytic enzyme is an isolated polypeptide having the amino acid sequence according to the mature polypeptide sequence in SEQ ID NO: 2 or an isolated polypeptide being at least 70% identical to said mature polypeptide sequence, wherein the anti staling amylase is preferably an isolated polypeptide according to SEQ ID NO: 9 or an isolated polypeptide being at least 70% identical thereto.

It has been surprisingly found that by applying the method according to the first aspect of the invention cakes are obtained with improved properties, among others an improved softness both initial and upon storage of the cake crumb (measured as decreased hardness of the cake crumb both initial and upon storage), and therefore an improved shelf life. Preferably the effect shown by the baking composition according to the invention in the method of the first aspect is improved or further improved if compared to the effect shown by adding the lipolytic enzyme alone or if compared to adding the anti staling amylase alone.

The method of the first aspect of the invention can be applied to all types of cakes, including shortened cakes, such as for example pound cake and butter cake, and including foam cakes, such as for example sponge cake, roulade, genoise and chiffon cake.

The method according to the first aspect of the invention starts by: a) preparing a cake batter starting from cake batter ingredients.

The basic ingredients of a cake are flour, eggs and sugar, therefore the cake batter ingredients may comprise at least flour, eggs and sugar. Optionally, leavening (generally baking powder), salt, water, emulsifiers (such as for example PGE's and monoglycerides), shortening, margarine, butter and/or oil are added (for example for pound cakes and muffins). Optionally, flavouring agents such as vanilla extract, cocoa powder or yeast extracts can be added. Also components to improve water binding such as hydrocolloids or (modified) starch can be used. The amount and type of ingredients largely varies depending on the type of cake to be produced.

Sponge cake is a type of soft cake based on wheat flour, sugar, baking powder and eggs (and optionally baking powder). The only fat present is from the egg yolk, which is sometimes added separately from the white. It is often used as a base for other types of cakes and desserts. A basic sponge cake is made by beating the eggs with sugar until they are light and creamy, then carefully sieving and folding in the flour (which may be mixed with a small amount of baking powder, although the air incorporated into the egg mixture can be sufficient for a good rise). Sometimes, the yolks are beaten with the sugar first while the whites are beaten separately, to be mixed in later. Modern practice frequently makes use of emulsifiers, such as polyglycerol esters and distilled monoglycerides, to bring further tolerance to the whipping of the batter and to permit the complete mix of ingredients to be blended together using the so-called "all-in-one method" or, in other words, to circumvent the need for whipping egg separately before incorporating the flour of the mixture. The mixture is then poured into the chosen cake tin and baked. Before the mixture has cooled, after cooking, it is still flexible. This allows the creation of such varieties as the Swiss roll.

A pound cake is traditionally prepared of one pound each of flour, butter, eggs, and sugar, optionally complemented with baking powder. Variations to this basic recipe are possible.

In chiffon cake the butter/margarine has been replaced by oil. Sugar and egg yolk content has been decreased compared to pound or sponge cake and egg white content has been increased.

There are several methods to combine cake ingredients, for example:
- Creaming method - butter and sugar are creamed together before the rest of the ingredients are gradually added.
- Melt-and-mix - dry ingredients are mixed together and then melted butter and other liquids are added to complete the cake.
- 'All-in-together' - the dry ingredients and shortening are placed in the food processor and liquid is gradually added.
- Sponge cake production - eggs and sugar are whipped to a froth and flour is carefully mixed in. No fat is used in this method.

When all the cake ingredients are mixed, the mixture is called cake batter. The method of the first aspect of the invention further comprises the step of: b) baking the cake batter to yield the cake.

Once the cake batter is ready, it may be placed in a suitable container and baked. The baking temperature and baking time will depend on the type of oven used and the type of cake to be produced. Those skilled in the art know how to choose the baking conditions in order to produce a cake of good quality.

In the method according to the first aspect of the invention an effective amount of a baking composition comprising a lipolytic enzyme and an anti staling amylase is added during the preparation of the cake batter.

The lipolytic enzyme present in the baking composition of the invention is an isolated polypeptide having the amino acid sequence according to the mature polypeptide sequence in SEQ ID NO: 2 or an isolated polypeptide being at least 70% identical to said mature polypeptide sequence.

The lipolytic enzyme present in the baking composition of the invention has been described in WO2009/106575 (the mature polypeptide is indicated in WO2009/106575 as amino acids 34-304 of SEQ ID NO: 2 and is encoded by the DNA sequence shown in SEQ ID NO: 1 in the present specification corresponding to SEQ ID NO: 1 in WO2009/106575) which is herewith incorporated by reference in its entirety.

In the context of the present invention "mature polypeptide" is defined herein as a polypeptide having lipolytic activity that is in its final form following translation and any post-translational modifications, such as N-terminal processing, C-terminal truncation, glycosylation, phosphorylation, etc. The process of maturation may depend on the particular expression vector used, the expression host and the production process. Preferably, the mature polypeptide is amino acids 34 to 304 in the amino acid sequence according to SEQ ID NO: 2. The mature polypeptide in the amino acid sequence according to SEQ ID NO: 2 is indicated as L01 in the sequence listing.

The lipolytic enzyme present in the baking composition of the invention used according to the present invention may comprise hydrolytic activity towards triacylglycerol lipids (according to EC3.1.1.3) and/or towards phospholipids (according to EC3.1.1.32 or according to EC3.1.1.4), and/or towards galactolipids (according to EC3.1.1.26). Preferably the lipolytic enzyme comprises hydrolytic activity towards triacylglycerol lipids (according to EC3.1.1.3), towards phospholipids (according to EC3.1.1.32 or according to EC3.1.1.4) and towards galactolipids (according to EC3.1.1.26).

Triacylglycerol lipids (also known as triglycerides or triacylglycerides) are mostly present in vegetable oils and animal fat. Lipases (EC 3.1.1.3) are defined herein as enzymes that hydrolyse one or more of the fatty acids from lipids, more specifically they hydrolyse the ester bond between fatty acid and hydroxyl groups of the glycerol.

Glycolipids (e.g. galactolipids) consist of a glycerol backbone with two esterified fatty acids in an outer (sn-1) and middle (sn-2) position, while the third hydroxyl group is bound to sugar residues such as in case of galactolipids a galactose, for example monogalactosyldiglyceride (MGDG) or digalactosyldiglyceride (DGDG). Galactolipase (EC 3.1.1.26) catalyses the hydrolysis of one or both fatty acyl group(s) in the sn-1 and sn-2 positions respectively from a galactosyldiglyceride into a galactosylmonoglyceride, for example monogalactosylmonoglyceride (MGMG) or digalactosylmonoglyceride (DGMG).

Phospholipids consist of a glycerol backbone with two esterified fatty acids in an outer (sn-1) and the middle (sn-2) position, while the third hydroxyl group of the glycerol is esterified with phosphoric acid. The phosphoric acid may, in turn, be esterified to for example an amino alcohol like ethanolamine (phosphatidylethanolamine), choline (phosphatidylcholine). Phospholipases are defined herein as enzymes that participate in the hydrolysis of one or more bonds in the phospholipids.

Several types of phospholipase activity can be distinguished which hydrolyse the ester bond(s) that link the fatty acyl moieties to the glycerol backbone: Phospholipase A1 (EC 3.1.1.32) and A2 (EC 3.1.1.4) catalyse the deacylation of one fatty acyl group in the sn-1 and sn-2 positions respectively, from a diacylglycerophospholipid to produce a lysophospholipid. Lysophospholipase (EC 3.1.1.5 - also called phospholipase B by the Nomenclature Committee of the International Union of Biochemistry and Molecular Biology (Enzyme Nomenclature, Academic Press, New York, 1992)) catalyses the hydrolysis of the remaining fatty acyl group in a lysophospholipid.

Preferably the determination of lipolytic activity towards triacylglycerol lipids is determined in an assay with the chromogenic substrate p-nitrophenyl palmitate as outlined in the Experimental section.

Preferably the determination of lipolytic activity towards galactolipids is determined using digalactosyl diglyceride (DGDG) as a substrate according to the procedure outlined in the Experimental section.

Preferably the determination of lipolytic activity towards phospholipids (either A1 or A2) is determined spectrophotometrically by using 1,2-dimercaptodioctanoyl-phosphatidylcholine as a substrate according to the procedure outlined in the Experimental section.

For the purpose of this invention, it is defined here that in order to determine the percent identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The identity is the percentage of identical matches between the two sequences over the reported aligned region.

A comparison of sequences and determination of percent identity between two sequences can be accomplished using a mathematical algorithm. The skilled person will be aware of the fact that several different computer programs are available to align two sequences and determine the homology between two sequences (Kruskal, J. B. (1983) An overview of squence comparison In D. Sankoff and J. B. Kruskal, (ed.), Time warps, string edits and macromolecules: the theory and practice of sequence comparison, pp. 1-44 Addison Wesley). The percent identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice,P. Longden,I. and Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap-open penalty of 10 and a gap extension penalty of 0.5. The skilled person will appreciate that all these different parameters will yield slightly different results but that the overall percentage identity of two sequences is not significantly altered when using different algorithms.

After alignment by the program NEEDLE as described above the percentage of identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, word length = 12 to obtain nucleotide sequences homologous to nucleic acid molecules of the invention. BLAST protein searches can be performed with the XBLAST program, score = 50, word length = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., XBLAST and NBLAST) can be used. See the homepage of the National Center for Biotechnology Information at http://www.ncbi.nlm.nih.gov/.

The baking enzyme composition according to the invention comprises a lipolytic enzyme which is an isolated polypeptide having lipolytic activity comprising the mature polypeptide in the amino acid sequence according to SEQ ID NO: 2, preferably comprising amino acids 34-304 of SEQ ID NO: 2 or an isolated polypeptide being at least 70% identical to said mature polypeptide sequence.

The lipolytic enzyme used in the method of the first aspect of the invention may be an isolated polypeptide having at least 70%, 75%, 80%, 85%, preferably at least 90%, 91%, 92%, 93%, 94%, 95%, 96%, 97%, 98% or 99% identity to the mature polypeptide in the amino acid sequence of SEQ ID NO: 2 and that exhibits at least one activity of the mature polypeptide in the amino acid sequence according to SEQ ID NO: 2. Therefore the isolated polypeptide having at least 70% identity to the mature polypeptide in the amino acid sequence of SEQ ID NO: 2 may comprise hydrolytic activity towards triacylglycerol lipids (according to EC3.1.1.3) and/or towards phospholipids (according to EC3.1.1.32 or according to EC3.1.1.4), and/or towards galactolipids (according to EC3.1.1.26). Preferably the lipolytic enzyme comprises hydrolytic activity towards triacylglycerol lipids (according to EC3.1.1.3), towards phospholipids (according to EC3.1.1.32 or according to EC3.1.1.4) and towards galactolipids (according to EC3.1.1.26).

The polypeptide having at least 70% identity to the mature polypeptide in the amino acid sequence of SEQ ID NO: 2 may contain substitutions of one or more amino acids of the mature polypeptide of the amino acid sequence according to SEQ ID NO: 2 or substitutions, insertions or deletions of amino acids which do not affect the particular functionality of the enzyme. Accordingly, a functionally neutral amino acid substitution is a substitution in the mature polypeptide of the amino acid sequence according to SEQ ID NO: 2 that does not substantially alters its particular functionality. For example, amino acid residues that are conserved among the proteins of the present invention are predicted to be particularly unameanable to alteration. Furthermore, amino acids conserved among the proteins according to the present invention and other lipolytic enzymes are not likely to be amenable to alteration.

In one embodiment the isolated polypeptide having at least 70% identity to the mature polypeptide in the amino acid sequence SEQ ID NO: 2 is the isolated polypeptide having an amino acid sequence according to the mature polypeptide in the amino acid sequence according to SEQ ID NO: 4 (indicated hereafter as L02), in another embodiment it is the isolated polypeptide having an amino acid sequence according to the mature polypeptide in the amino acid sequence according to SEQ ID NO: 6 (indicated hereafter as L03), and in yet another embodiment it is the isolated polypeptide having an amino acid sequence according to the mature polypeptide in the amino acid sequence according to SEQ ID NO: 8 (indicated hereafter as L04). In a preferred embodiment the mature polypeptide in the amino acid sequence according to SEQ ID NO: 4, SEQ ID NO: 6 or SEQ ID NO: 8 respectively is amino acid sequence 34 to 304 in the amino acid sequence according to SEQ ID NO: 4, SEQ ID NO: 6 or SEQ ID NO: 8, respectively. The lipolytic enzymes according to SEQ ID NO: 4, 6, 8 have been described in WO2009/106575 (the mature lipolytic enzymes according to SEQ ID NO: 4, 6, 8 in the present specification correspond to amino acid 34 to 304 in the amino acid sequence SEQ ID NO: 4, 6, 8 of WO2009/106575, respectively and are encoded by the polynucleotide according to SEQ ID 3, 5, 7 of the present patent specification, corresponding to SEQ ID NO: 3, 5, 7 in WO2009/106575).

The word "polypeptide" (or protein or enzyme) is used herein for chains containing more than seven amino acid residues. All oligopeptide and polypeptide formulas or sequences herein are written from left to right and in the direction from amino terminus to carboxy terminus. The one-letter code of amino acids used herein is commonly known in the art and can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989).

By "isolated" polypeptide or protein or enzyme is intended a polypeptide or protein removed from its native environment. For example, recombinantly produced polypeptides and proteins produced in host cells are considered isolated for the purpose of the invention as are native or recombinant polypeptides which have been substantially purified by any suitable technique such as, for example, the single-step purification method disclosed in Smith and Johnson, Gene 67: 31-40 (1988).

The baking composition used in the method of the invention further comprises an anti staling amylase, preferably the anti staling amylase is an isolated polypeptide according to SEQ ID NO: 9 or an isolated polypeptide being at least 70% identical thereto.

The anti staling amylase is an enzyme capable of retarding crumb firming of the cake when used in effective amount in the batter ingredients. The anti staling effect can be measured as a reduced decrease in crumb softness upon storage of the cake over time (or a reduced increase in hardness of the cake crumb over time) if compared with a cake where no anti staling amylase has been added. Preferably the anti staling amylase is a maltogenic α-amylase. The maltogenic α-amylase is an enzyme classified in EC 3.2.1.133. The primary enzymatic activity of the maltogenic α-amylase results in the degradation of amylopectin and amylose to maltose in the alpha configuration and longer maltodextrins. A maltogenic α-amylase may also hydrolyse maltotriose and cyclodextrins. The enzymatic activity of the maltogenic α-amylase does not require a non-reducing end on the substrate.

In a preferred embodiment of the invention the anti staling amylase is the maltogenic α-amylase corresponding to the isolated polypeptide depicted in SEQ ID NO: 9. The polypeptide depicted in SEQ ID NO: 9 is the maltogenic α-amylase known under the name Novamyl® (Novozymes, Denmark), which coding DNA is derived from the *Bacillus* strain NCIB 11837 and described e.g. in WO91/04669. SEQ ID NO: 9 and its coding DNA sequence are shown in WO99/43793: SEQ ID NO: 9 corresponds to amino acids 1-686 of SEQ ID NO: 1 of WO99/43793 and the gene coding for SEQ ID NO: 9 is the nucleic acid sequence set forth in SEQ ID NO: 1 of WO99/43793.

Preferably the maltogenic α-amylase activity is determined according to the MANU method described in the Experimental section of this application or of WO99/43793.

In one embodiment of the invention the isolated polypeptide having anti staling amylase activity, preferably maltogenic α-amylase activity, is an isolated polypeptide being at least 70% identical to SEQ ID NO: 9, preferably being at least 75%, 80%, 85%, at least 90%, 91%, 92%, 93%, 94%, 95%, 96%, 97%, 98% or 99% identical thereto.

In one embodiment of the invention the isolated polypeptide being at least 70% identical to SEQ ID NO: 9 has the ability to form cyclodextrin when acting on starch and is a maltogenic α-amylase variant, e.g. a Novamyl® variant as described in WO99/43793, which is herewith incorporated by reference in its entirety. In one embodiment the Novamyl® variant includes, if compared with SEQ ID NO: 9, a deletion in the region 190-195 in SEQ ID NO: 9, preferably a deletion of amino acid 191 to 195 (indicated hereafter as Δ (191-195)) and/or a substitution of amino acid 188 and/or 189, e.g. F188L and/or T189Y. In an embodiment the isolated polypeptide having anti staling amylase activity being at least 70% identical to SEQ ID NO: 9, preferably being at least 75%, 80%, 85%, at least 90%, 91%, 92%, 93%, 94%, 95%, 96%, 97%, 98% or 99% identical thereto comprises an amino acid modification compared to SEQ ID NO: 9 at a position that corresponds to residue F188, such as F188I or F188L.

In another embodiment of the invention the isolated polypeptide being at least 70% identical to SEQ ID NO: 9 is a maltogenic α-amylase variant with improved ability to reduce retrogradation of starch and/or staling of bread as described in WO99/43794 which is herewith incorporated by reference in its entirety. Preferred variants comprise a modification at one or more positions corresponding to the following amino acid residues in SEQ ID NO: 9: A30, K40, N115, T142, F188, T189, P191, A192, G193, F194, S195, D261, N327, K425, K520 and N595, more preferably the variant comprises one or more modifications corresponding to the following amino acids in SEQ ID NO: 9: A30D, K40R, N115D, T142A, F188L, T189Y, Δ (191-195), D261G, N327S, K425E, K520R and N595I.

In preferred embodiment the isolated polypeptide being at least 70% identical to SEQ ID NO: 9 is a maltogenic α-amylase variant having higher tolerance for sucrose if compared to the polypeptide of SEQ ID NO: 9, as described in WO2006/032281 which is herewith incorporated by reference in its entirety. Preferably the anti staling amylase is a polypeptide being at least 70% identical to SEQ ID NO: 9 and has an amino acid sequence which compared with SEQ ID NO: 9 has one or more amino acid alterations which is a substitution, a deletion or an insertion at one of the a position I15, R18, K44, N86, T87, G88, Y89, H90, Y92, W93, F188, T189, D190, P191 , A192, F194, L196, D329, N371 , D372, P373, N375 or R376 preferably a substitution, a deletion or an insertion at one of the a position N86T/VG/K, T87N/Q, H90W/F/Y/R/M, F188I/L/T/G/V, D190G, a deletion at position P191 (indicated hereafter as P191*), A192G/Q/R, F194S/L/Y, L196F, L225S, F252L, D261G, T288P, I290V, N371 K/R/F/Y/Q, D372E/Q/S/T/A/V, N375S, S446A, G469R, S578G or P642Q.

In preferred embodiments the isolated polypeptide having at least 70% identity to SEQ ID NO: 9 has an amino acid sequence which compared with SEQ ID NO: 9 comprises or has the following combinations of amino acid alterations:
D261 G + T288P
F188L + D261G + T288P
T288P
Y89F + D261 G + T288P
N86V + F188L + D261G + T288P
Y89F + F188L + D261G + T288P
Y89H + F188L + D261G + T288P
N86T + F188L + D261 G + T288P
F194S + D261G + T288P
L196F
D261 G + T288P + D372V
Q184H + N187D + F194Y
D190G
N86G + Y89M + F188L + D261 G + T288P
F188L + D190G + D261G + T288P
A192Q + D261 G + T288P + S446A
F188H
P191*
A192*
A192* + G193*
An insertion of an A at position 192 (indicated hereafter as *192aA)
N86K + F252L + D261 G + T288P
F194Y + L225S + 261 G + T288P
F194L + D261 G + T288P
F194S+ D261 G + T288P + P642Q
D261 G + T288P + N375S
F188T
F188G
F188V
A192R + F194L + D261 G + T288P + G469R
A192G + D261 G + T288P
Y89F + D261 G + T288P + I290V + N375S.

In another embodiment the isolated polypeptide having anti staling amylase activity being at least 70% identical, preferably being at least 75%, 80%, 85%, at least 90%, 91%, 92%, 93%, 94%, 95%, 96%, 97%, 98% or 99% identical thereto, comprises the combination of amino acid alterations F188L+D261 G+T288P compared to SEQ ID NO: 9.

In another preferred embodiment the isolated polypeptide being at least 70% identical to SEQ ID NO: 9 is a maltogenic α-amylase variant having higher tolerance for sucrose if compared to the polypeptide of SEQ ID NO: 9, as described in WO2008/148845 which is herewith incorporated by reference in its entirety. Preferably the anti staling amylase is a polypeptide being at least 70% identical to SEQ ID NO: 9 comprises the two substitutions D261 G and T288P and at least one additional amino acid alteration which compared to SEQ ID NO:9 is a substitution, a deletion or an insertion at position Y89, W93, P191, F194, Y360 or N375.

In preferred embodiments the isolated polypeptide having at least 70% identity to SEQ ID NO: 9 comprises or has an amino acid sequence which compared with SEQ ID NO: 9 has one of the following sets of alterations:
F194Y + D261G + T288P + N375S
I15T + P191S + D261G + T288P + N375S + S640I
Y89F + P191 S + D261 G + T288P
I15T + Y89F + P191 S + D261G + T288P
Y89F + F194Y + D261G + T288P
Y89F + D261G + T288P + N375S
Y89F + P191S + F194Y + D261G + T288P
Y89F + P191S + D261G + T288P + N375S
Y89F + P191S + D261G + T288P + Y360N
Y89F + P191S + D261G + T288P + Y360F
Y89F + W93Y + P191S + D261G + T288P
Y89F + W93F + P191S + D261G + T288P
Y89F + P191S + F194Y + D261G + T288P + N375S

In a preferred embodiment of the baking composition according to the invention to be used in the method to produce cake according to the invention the lipolytic enzyme is the isolated polypeptide according to the mature peptide in SEQ ID NO: 2 and the anti staling amylase is the maltogenic α-amylase according to SEQ ID NO: 9 or an isolated polypeptide being at least 70% identical thereto and which as been described in any of the preceding embodiments of the invention.

In a second aspect, the invention relates to the use of a baking composition comprising a lipolytic enzyme and an anti staling amylase or of a cake pre-mix comprising flour and a baking composition comprising a lipolytic enzyme and an anti staling amylase in the production of cake to improve one or more batter or cake properties, and/or to allow the reduction of eggs and/or fat in the cake, wherein the lipolytic enzyme is an isolated polypeptide having the amino acid sequence according to the mature polypeptide sequence in SEQ ID NO: 2 or an isolated polypeptide being at least 70% identical to said mature polypeptide sequence and wherein the anti staling amylase is preferably an isolated polypeptide according to SEQ ID NO: 9 or an isolated polypeptide being at least 70% identical thereto.

The lipolytic enzyme, the anti staling amylase and the baking composition comprising the same are those as defined in the first aspect of the invention and in the section "Preferred Embodiments" of this patent specification.

A baking composition according to the invention may be used in the production of cake to improve one or more batter or cake properties and/or to allow the reduction of eggs and/or fat in the cake.

In a preferred embodiment of the invention the baking composition according to the invention may be used in a method according to the first aspect or the use according to the second aspect wherein the baking composition or a cake pre-mix comprising flour and the baking composition according to the invention can be used to improve at least one property selected from (i) initial crumb softness, (ii) crumb pore homogeneity, (iii) crumb pore diameter, (iv) crumb softness upon storage, (v) shelf life, (vi) cohesiveness and/or (vii) cake volume in the cake.

In another preferred embodiment of the invention the baking composition according to the invention can be used in a method according to the first aspect of the invention or in the use according to the second aspect of the invention wherein the baking composition or a cake pre-mix comprising flour and the baking composition can be used to improve at least one of the properties selected from (i) batter viscosity, (ii) specific density in the cake batter.

In yet another preferred embodiment of the invention the baking composition according to the invention can be used in a method according to the first aspect or the use according to the second aspect of the invention wherein the baking composition or a cake pre-mix comprising flour and the baking composition can be used to reduce the amount of eggs and/or fat used in the recipe whilst at least maintaining at least one of the properties selected from the group consisting of: (i) batter viscosity, (ii) specific density, (iii) initial crumb softness, (iv) crumb pore homogeneity, (v) crumb pore diameter, (vi) crumb softness upon storage, (vii) shelf life, (viii) cohesiveness and/or (ix) cake volume.

Preferably the effect shown by the baking composition according to the invention in the use of the second aspect is improved or further improved if compared to the effect shown by adding the lipolytic enzyme alone or if compared to adding the anti staling amylase alone.

The term at least maintaining is hereby used to indicate that a property is maintained or improved.

Measuring whether a property is maintained, improved or deteriorated in general is measured by preparing a batter and/or a cake in an original recipe, not containing the baking composition and another batter and/or cake in a recipe containing the baking composition and optionally less eggs and/or fat and comparing a certain property. In case the properties of both are substantially the same, the property is maintained, in case they differ either an improvement or a deterioration has taken place. For all mentioned properties below a measurement method has been given as well as an indication when a property can be considered as improved.

The batter viscosity can be measured with a Farinograph by standard methods according to the International Association of Cereal Chemistry (ICC) and the American Association of Cereal Chemistry (AACC 54-2, ICC 115).

Whether the batter viscosity has improved or deteriorated can for example be measured by comparing the batter prepared with the baking composition, either containing or not containing a reduced amount of eggs and/or fat, to a batter prepared without the baking composition according to the invention. In case the batter viscosity is the same for both batters, it has been maintained. In case the batter viscosity has increased, it has improved.

The specific density can be measured by weighing a predetermined volume of batter. The specific density is improved if it is decreased.

The crumb softness of the cake is evaluated either empirically by the skilled test baker or measured by the use of a texture analyzer (TA-XT2 or TA-XTplus from Stable Micro Systems, UK) as known in the art. Actually crumb hardness is measured as is known to the person skilled in the art. Crumb hardness is the opposite of crumb softness. Generally for products such as cakes, a 4 cm cylindrical probe (P36) is used to asses crumb hardness and other characteristics. In the method used in the present invention the sample size was bigger than the diameter of the probe and cake slices of 2cm width were subjected to two compression cycles using the texture analysis probe. For each compression cycle the probe was pushed into the cake sample at a rate of 2mm/sec for 5 sec and then withdrawn at the same rate; there was a 5sec pause between the two compression cycles. Crumb hardness was defined as being the maximum force registered during the first compression cycle.

The crumb softness measured within 24 hours after baking (actually measured as crumb hardness within 24 hours) is called initial crumb softness. The crumb softness more than 24 hours after baking (actually measured as crumb hardness more than 24 hours after baking) is called crumb softness upon storage, and is also a measure for determining shelf life. In case the initial crumb softness has increased, it has improved. In case the crumb softness upon storage has increased, it has improved.

Crumb pore homogeneity can be evaluated empirically by the skilled test baker or by digital image analysis as known in the art (e.g. C-cell, Calibre Control International Ltd, Appleton, Warrington, UK). In case the deviation in pore size is small, the crumb is called more homogeneous. In case the deviation in pore size has become smaller, the property is improved.

Crumb pore diameter can be evaluated using digital image analysis as known in the art (e.g. C-cell, Calibre Control International Ltd, Appleton, Warrington, UK). In case the average crumb pore diameter decreases, the property is improved. Preferably, this is the case when at the same time the same cake volume is maintained.

The shelf-life of the cake can be measured by determining the resilience of the cake in time. This is part of the method to measure crumb softness, as is known to the person skilled in the art, whereby the relaxation of the cake is also measured by the use of a texture analyzer (TA-XT2 or TA-XTplus from Stable Micro Systems,UK) as known in the art.

Cohesiveness of the cake can be measured with a texture analyzer as indicated above and as known in the art. It is a measure of how well the cake withstands a second deformation relative to how it behaved under the first deformation.

The volume of a given cake can be determined by an automated bread volume analyser (eg. BVM-3, TexVol Instruments AB, Viken, Sweden), using ultrasound or laser detection as known in the art. In case the volume is increased, the property is improved. Alternatively the cake height after baking in the same size tin is an indication of the cake volume. In case the cake height is increased, the cake volume has increased.

The baking composition according to the invention or the pre-mix according to the invention is added in an effective amount in the method of the first aspect or in the use of the second aspect.

In a preferred embodiment of the method of the first aspect or in the use of the second aspect of the invention the lipolytic enzyme is added in an amount of 10 - 2000 DLU/kg of cake batter, preferably in an amount of 50 - 600DLU/kg of cake batter.

In a preferred embodiment of the method of the first aspect or in the use of the second aspect of the invention the anti staling amylase is added in an amount of 250 - 6000 MANU/kg of cake batter, preferably in an amount of 500 - 3000 MANU /kg of cake batter.

In yet another embodiment of the invention the method of the first aspect or in the use of the second aspect of the invention the cake comprises a reduced amount of eggs and/or fat if compared with a conventional cake. The amount of eggs and/or fat present in a conventional cake may vary and will depend on the type of cake.

The reduction of the amount of eggs and/or fat which is possible according to the present invention differs per type of cake. The man skilled in the art knows the amount of eggs and/or fat which are regularly present in cake recipes. In general a reduction of the amount of eggs of at least 5% w/w can be reached. More preferably a reduction of the amount of eggs of at least 10% w/w can be reached, even more preferably a reduction of at least 15% w/w can be reached. It was shown that even a reduction of the amount of eggs used of at least 20% w/w can be reached. The reduction of the amount of eggs can be at least 30%w/w, 40% w/w or even at least 50% w/w.

In cake recipes eggs provide natural emulsifiers as well as egg protein. Egg protein is important for froth formation in the batter and for the cake cohesiveness. In cake recipes wherein the amount of eggs has been reduced, especially if reduced of at least 30% w/w, 40% w/w or 50% w/w, the loss of egg protein can (partially) be compensated by the addition of other protein sources and/or hydrocolloids. Examples of protein sources are whey protein, soy protein, modified wheat protein, albumin, etcetera. Examples of hydrocolloids are guar gum, alginate, pectin, xanthan gum, etcetera. Therefore in one embodiment of the invention one or more protein sources and/or one or more hydrocolloids are used in the cake recipe to replace the protein content present in the eggs removed.

In general a reduction of the amount of fat of at least 10% can be reached. More preferably a reduction of the amount of fat of at least 20% can be reached, even more preferably a reduction of at least 30% can be reached. It was shown that even a reduction of the amount of fat used of at least 50% can be reached.

In one embodiment of the method of the first aspect the invention or of the use of the second aspect, modified starch can be used to reduce the amount of fat used in the recipe even further. All types of modified starch can be used, for example modified potato starch or modified wheat starch. Preferably modified potato starch is used, such as for example disclosed in US 6,864,063. Most preferably modified potato starch is used which is obtained by treating potato starch with amylomaltase, more preferably with amylomaltase derived from *Bacillus amyloliquefaciens.* An example of modified potato starch obtained by treating potato starch with amylomaltase derived from *Bacillus amyloliquefaciens* is sold under the trademark Etenia^{®} (Avebe Food).

In one embodiment of the method of the first aspect or of the use of the second aspect the baking composition comprising the lipolytic enzyme and the anti staling amylase according to the invention further comprises one or more additional enzymes. The additional enzyme may be of any origin, including mammalian and plant, and preferably of microbial (bacterial, yeast or fungal) origin and may be obtained by techniques conventionally used in the art.

In a preferred embodiment, the additional enzyme may be an amylase, such as an alpha-amylase (useful for providing sugars fermentable by yeast and retarding staling), beta-amylase, maltogenic amylase or non-maltogenic amylase, a cyclodextrin glucanotransferase, a protease, a peptidase, in particular, an exopeptidase (useful in flavour enhancement), transglutaminase, lipase (useful for the modification of lipids present in the batter), galactolipase, phospholipase, cellulase, hemicellulase, in particular a pentosanase such as xylanase (useful for the partial hydrolysis of pentosans, more specifically arabinoxylan), protease (useful for gluten weakening in particular when using hard wheat flour), protein disulfide isomerase, e.g., a protein disulfide isomerase as disclosed in WO 95/00636, glycosyltransferase, peroxidase, laccase, or oxidase, hexose oxidase, e.g., a glucose oxidase, aldose oxidase, pyranose oxidase, lipoxygenase or L-amino acid oxidase.

When one or more additional enzyme activities are to be added in accordance with the method or use of the present invention, these activities may be added separately or together with lipolytic enzyme and the anti staling amylase. The other enzyme activities may be any of the enzymes described above and may be dosed in accordance with established baking practices.

In a preferred embodiment the baking composition according to the invention to be used in the method of the first aspect or the use of the second aspect further comprises one or more enzymes selected from a phospholipase, and a bacterial α-amylase or a combination thereof. In a preferred embodiment the phospholipase is added in an amount of 200-2000 EYU/kg of batter. In another preferred embodiment the bacterial α-amylase can be added to the batter in an amount of 2-20 RAU/kg batter.

In a third aspect the invention discloses the baking composition comprising a lipolytic enzyme and an anti staling amylase, wherein the lipolytic enzyme is an isolated polypeptide having the amino acid sequence according to the mature polypeptide sequence in SEQ ID NO: 2 or an isolated polypeptide being at least 70% identical to said mature polypeptide sequence, wherein the anti staling amylase is preferably an isolated polypeptide according to SEQ ID NO: 9 or an isolated polypeptide being at least 70% identical thereto. The preferred embodiments of the baking composition according to the third aspect of the invention have been described in the first and second aspect of the invention and in the "Preferred Embodiments" of this patent specification.

In a fourth aspect, the invention discloses a cake pre-mix comprising flour and a baking composition according to the invention. Cake mixes are often used at home because they are convenient. Most cake mixes simply require adding the package contents to eggs and oil in a bowl and mixing for two to three minutes. The mixture is then ready to be poured into pans and baked.

The cake pre-mixes may be used in a method of the first aspect or a use of the second aspect of the invention.

It has been further found that if a lipolytic enzyme as described herein is applied alone in a method to produce a cake it may have beneficial effects. Such as in a method to produce a cake comprising:
a) preparing a cake batter starting from cake batter ingredients,
b) baking the cake batter to yield the cake,
wherein in the preparation of the cake batter a baking composition comprising a lipolytic enzyme or a cake pre-mix comprising flour and a baking composition comprising a lipolytic enzyme is added in an effective amount,
wherein the lipolytic enzyme is an isolated polypeptide having the amino acid sequence according to the mature polypeptide sequence in SEQ ID NO: 2 or an isolated polypeptide being at least 70% identical to said mature polypeptide sequence,

it may result to improve volume and/or have anti-firming effect in all cake systems such as pound cake (including emulsifier-free or egg reduced), high ratio cake, muffins or sponge cakes. Furthermore the enzyme may be efficient in improving the cohesiveness of cakes, especially in pound cakes.

Therefore in a further aspect the invention discloses the use of a lipolytic enzyme which is an isolated polypeptide having the amino acid sequence according to the mature polypeptide sequence in SEQ ID NO: 2 or an isolated polypeptide being at least 70% identical to said mature polypeptide sequence to improve at least one cake property selected from: (i) volume, (ii) initial crumb softness, (iii) crumb softness upon storage and/or (iv) cohesiveness. In one embodiment of this aspect the cake is an egg and/or fat reduced cake, in another embodiment of this aspect the cake is an emulsifier free cake. Preferred embodiments relating to this aspect of the invention can be found in the section "Preferred embodiments" in this specification.

It has further been found that if an anti staling amylase as described herein is applied alone in a method to produce cake it may have beneficial effects. Such as in a method to produce cake comprising:
a) preparing a cake batter starting from cake batter ingredients,
b) baking the cake batter to yield the cake,
wherein in the preparation of the cake batter a baking composition comprising an anti staling amylase or a cake pre-mix comprising flour and a baking composition comprising an anti staling amylase is added in an effective amount,
wherein the anti staling amylase is preferably an isolated polypeptide according to SEQ ID NO: 9 or an isolated polypeptide being at least 70% identical thereto,

it may result in reduced loss of cohesiveness and resilience both initial and after several weeks and therefore in improved shelf life in all cake systems, including pound cake (including emulsifier-free or egg reduced), high ratio cake, muffins or sponge cakes. Furthermore the enzyme may be efficient in improving the softness of the crumb in cake, especially in pound cake and sponge cake and in some situations in improving the volume of the cake.

Therefore in yet a further aspect of the invention it is disclosed the use of an anti staling amylase, preferably a maltogenic α-amylase, more preferably an isolated polypeptide according to SEQ ID NO: 9 or an isolated polypeptide being at least 70% identical thereto to improve at least one cake property selected from: (i) initial crumb softness, (ii) crumb softness upon storage, (iii) shelf life, (iv) cohesiveness and/or (v) cake volume. In one embodiment of this aspect the cake is an egg reduced and/or fat reduced cake, in another embodiment of this aspect the cake is an emulsifier free cake. Preferred embodiments relating to this aspect of the invention can be found in the section "Preferred embodiments" in this specification.

Preferred or further embodiments of one aspect of the invention are applicable to other aspects of the invention.

### Preferred embodiments of the invention

1. A baking composition comprising a lipolytic enzyme and an anti staling amylase, wherein the lipolytic enzyme is an isolated polypeptide having the amino acid sequence according to the mature polypeptide sequence in SEQ ID NO: 2 or an isolated polypeptide being at least 70% identical to said mature polypeptide sequence, wherein the anti staling amylase is preferably an isolated polypeptide according to SEQ ID NO: 9 or an isolated polypeptide being at least 70% identical thereto.
2. A baking composition according to embodiment 1, wherein the lipolytic enzyme is the mature polypeptide sequence in SEQ ID NO: 2.
3. A baking composition according to embodiment 1-2, wherein the lipolytic enzyme is an isolated polypeptide having at least 70%, 75%, 80%, 85%, preferably at least 90%, 91%, 92%, 93%, 94%, 95%, 96%, 97%, 98% or 99% identity to the mature polypeptide in the amino acid sequence of SEQ ID NO: 2 and that exhibits at least one activity of the mature polypeptide in the amino acid sequence according to SEQ ID NO: 2.
4. A baking composition according to embodiment 3, wherein the isolated polypeptide being at least 70% identical to the mature polypeptide in SEQ ID NO: 2 is the mature polypeptide in SEQ ID NO: 4, 6 or 8.
5. A baking compositions according to embodiment 1-4, wherein the mature polypeptide in SEQ ID NO: 2, 4, 6 or 8 is amino acids 34 to 304 in the amino acid sequence according to SEQ ID NO: 2, 4, 6, or 8.
6. A baking composition according to embodiment 1-5 wherein the lipolytic enzyme comprises hydrolytic activity towards triacylglycerol lipids (according to EC3.1.1.3) and/or towards phospholipids (according to EC3.1.1.32 or according to EC3.1.1.4), and/or towards galactolipids (according to EC3.1.1.26), preferably the lipolytic enzyme comprises hydrolytic activity towards triacylglycerol lipids (according to EC3.1.1.3), towards phospholipids (according to EC3.1.1.32 or according to EC3.1.1.4) and towards galactolipids (according to EC3.1.1.26).
7. A baking composition according to embodiment 1-6 wherein the anti staling amylase is a maltogenic α-amylase, preferably an isolated polypeptide according to SEQ ID NO: 9.
8. A baking composition according to embodiment 1-7, wherein the anti staling amylase is an isolated polypeptide at least 70% to SEQ ID NO:9, preferably at least 75%, 80%, 85%, 90%, 91%, 92%, 93, 94%, 95%, 96%, 97%, 98% or 99% identical thereto.
9. A baking composition according to embodiment 8 wherein the isolated polypeptide being at least 70% identical to SEQ ID NO: 9 has the ability to form cyclodextrin when acting on starch and is a maltogenic α-amylase variant, preferably the maltogenic α-amylase variant includes, if compared with SEQ ID NO: 9, a deletion in the region 190-195 in SEQ ID NO: 9, preferably a deletion of amino acid 191 to 195 and/or a substitution of amino acid 188 and/or 189.
10. A baking composition according to embodiment 9 wherein the isolated polypeptide having anti staling amylase activity being at least 70% identical to SEQ ID NO: 9, preferably being at least 75%, 80%, 85%, at least 90%, 91%, 92%, 93%, 94%, 95%, 96%, 97%, 98% or 99% identical thereto comprises an amino acid modification compared to SEQ ID NO: 9 at a position that corresponds to residue F188, preferably that corresponds to F188I or F188L.
11. A baking composition according to embodiment 8 wherein the isolated polypeptide being at least 70% identical to SEQ ID NO: 9 comprises a modification at one or more positions corresponding to the amino acid residues in SEQ ID NO: 9: A30, K40, N115, T142, F188, T189, P191, A192, G193, F194, S195, D261, N327, K425, K520 and N595, more preferably the variant comprises one or more modifications corresponding to the amino acids in SEQ ID NO: 9: A30D, K40R, N115D, T142A, F188L, T189Y, Δ (191-195), D261G, N327S, K425E, K520R and N595I.
12. A baking composition according to embodiment 8 wherein the isolated polypeptide being at least 70% identical to SEQ ID NO: 9 has an amino acid sequence which compared with SEQ ID NO: 9 has one or more amino acid alterations which is a substitution, a deletion or an insertion at one of the a position I15, R18, K44, N86, T87, G88, Y89, H90, Y92, W93, F188, T189, D190, P191, A192, F194, L196, L225, F252, D261, T288, I290 D329, N371, D372, P373, N375 or R376, S446, G469, S578 or P642, preferably a substitution, a deletion or an insertion at one of the a position N86T/VG/K, T87N/Q, H90W/F/Y/R/M, F188I/L/T/G/V, D190G, P191*, A192G/Q/R, F194S/L/Y, L196F, L225S, F252L, D261G, T288P, I290V, N371 K/R/F/Y/Q, D372E/Q/S/T/A/V, N375S, S446A, G469R, S578G or P642Q.
13. A baking composition according to embodiment 12 wherein the isolated polypeptide having at least 70% identity to SEQ ID NO: 9 has an amino acid sequence which compared with SEQ ID NO: 9 has the following combinations of amino acid alterations:
   D261 G + T288P;
   F188L + D261G + T288P;
   T288P;
   Y89F + D261 G + T288P;
   N86V + F188L + D261G + T288P;
   Y89F + F188L + D261G + T288P;
   Y89H + F188L + D261G + T288P;
   N86T + F188L + D261G + T288P;
   F194S + D261G + T288P;
   L196F;
   D261G + T288P + D372V;
   Q184H + N187D + F194Y;
   D190G;
   N86G + Y89M + F188L + D261G + T288P;
   F188L + D190G + D261G+ T288P;
   A192Q + D261G + T288P + S446A;
   F188H;
   P191*;
   A192*;
   A192* + G193*;
   *192aA;
   N86K + F252L + D261G + T288P;
   F194Y + L225S + 261G + T288P;
   F194L + D261G + T288P;
   F194S+ D261G + T288P + P642Q;
   D261G + T288P + N375S;
   F188T;
   F188G;
   F188V;
   A192R + F194L + D261G+ T288P + G469R;
   A192G + D261G + T288P;
   Y89F + D261G + T288P + I290V + N375S.
14. A baking composition according to embodiment 13 wherein the isolated polypeptide having anti staling amylase activity being at least 70% identical, preferably being at least 75%, 80%, 85%, at least 90%, 91%, 92%, 93%, 94%, 95%, 96%, 97%, 98% or 99% identical thereto, comprises the combination of amino acid alterations F188L+D261G+T288P compared to SEQ ID NO: 9.
15. A baking composition according to embodiment 12-14, which comprises the two substitutions D261 G and T288P and at least one additional amino acid alteration which is a substitution, a deletion or an insertion at position Y89, W93, P191, F194, Y360 or N375.
16. A baking composition according to embodiment 15 wherein the isolated polypeptide having at least 70% identity to SEQ ID NO: 9 has an amino acid sequence which compared with SEQ ID NO: 9 has one of the following sets of alterations:
   F194Y + D261G + T288P + N375S;
   I15T + P191S + D261G + T288P + N375S + S640I;
   Y89F + P191S + D261G + T288P;
   I15T + Y89F + P191S + D261G + T288P;
   Y89F + F194Y + D261G + T288P;
   Y89F + D261G + T288P + N375S;
   Y89F + P191S + F194Y + D261G + T288P;
   Y89F + P191S + D261G + T288P + N375S;
   Y89F + P191S + D261G + T288P + Y360N;
   Y89F + P191S + D261G + T288P + Y360F;
   Y89F + W93Y + P191S + D261G + T288P;
   Y89F + W93F + P191S + D261G + T288P;
   Y89F + P191S + F194Y + D261G + T288P + N375S.
17. A baking composition according to embodiment 1-16 which further comprise one or more further enzymes, preferably further comprises one or more additional enzymes, preferably selected from a phospholipase, and a bacterial α-amylase or a combination thereof.
18. A cake pre-mix comprising flour and a baking composition according to embodiment 1-17.
19. Method to produce a cake comprising:
   a) preparing a cake batter starting from cake batter ingredients,
   b) baking the cake batter to yield the cake
      wherein in the preparation of the cake batter a baking composition comprising a lipolytic enzyme and an anti staling amylase according to embodiment 1-17 or a cake pre-mix according to embodiment 18 is added in an effective amount.
20. Use of a baking composition comprising a lipolytic enzyme and an anti staling amylase according to embodiment 1-17 or a cake pre-mix according to embodiment 18 in the production of cake to improve one or more batter or cake properties, and/or to allow the reduction of eggs and/or fat in the cake.
21. The method according to embodiment 19 or the use according to embodiment 20 wherein the baking composition according to embodiment 1-17 or a cake pre-mix according to embodiment 18 can be used to improve at least one property selected from (i) initial crumb softness, (ii) crumb pore homogeneity, (iii) crumb pore diameter, (iv) crumb softness upon storage, (v) shelf life, (vi) cohesiveness and/or (vii) cake volume in the cake.
22. The method according to embodiment 19 or 21 or the use according to embodiment 20-21 wherein the baking composition according to embodiment 1-17 or a cake pre-mix according to embodiment 18 can be used to improve at least one of the properties selected from (i) batter viscosity, (ii) specific density in the cake batter.
23. The method according to embodiment 19 or 21-22 or the use according to embodiment 20-22 wherein the baking composition according to embodiment 1-17 or a cake pre-mix according to embodiment 18 can be used to reduce the amount of eggs and/or fat used in the recipe whilst at least maintaining at least one of the properties selected from the group consisting of: (i) batter viscosity, (ii) specific density, (iii) initial crumb softness, (iv) crumb pore homogeneity, (v) crumb pore diameter, (vi) crumb softness upon storage, (vii) shelf life, (viii) cohesiveness and/or (ix) cake volume.
24. The method according to embodiment 19 or 21-23 or the use according to embodiment 20-23 wherein the lipolytic enzyme is added in an amount of 10 - 2000 DLU/kg of cake batter, preferably in an amount of 50 - 600DLU/kg of cake batter.
25. The method according to embodiment 19 or 21-24 or the use according to embodiment 20-24 wherein the anti staling amylase is added in an amount of 250 -6000 MANU/kg of cake batter, preferably in an amount of 500 - 3000 MANU /kg of cake batter.
26. The method according to embodiment 19 or 21-25 or the use according to embodiment 20-25 wherein the cake comprises a reduced amount of eggs and/or fat if compared with a conventional cake.
27. The method or use according to embodiment 26 wherein the amount of eggs is reduced with at least 5% w/w, preferably with at least 10% w/w, more preferably with at least 15% w/w, most preferably the amount of eggs is reduced with at least 20% w/w, 30% w/w, 40% w/w, 50% w/w.
28. The method or use according to embodiment 26-27 wherein the amount of fat is reduced with at least 10% w/w, preferably the amount of fat is reduced with at least 20% w/w, even more preferably the amount of fat is reduced with at least 30% w/w.
29. The method or use according to embodiment 26-28 wherein the cake ingredients further comprise modified starch, preferably modified potato starch is used which is obtained by treating potato starch with amylomaltase, more preferably with amylomaltase derived from *Bacillus amyloliquefaciens.*
30. Use of a lipolytic enzyme which is an isolated polypeptide having the amino acid sequence according to the mature polypeptide sequence in SEQ ID NO: 2 or an isolated polypeptide being at least 70% identical to said mature polypeptide sequence to improve at least one cake property selected from: (i) volume, (ii) initial crumb softness, (iii) crumb softness upon storage and/or (iv) cohesiveness.
31. The use according to embodiment 30, wherein the lipolytic enzyme is the mature polypeptide sequence in SEQ ID NO: 2.
32. The use according to embodiments 30-31, wherein the lipolytic enzyme is an isolated polypeptide having at least 70%, 75%, 80%, 85%, preferably at least 90%, 91 %, 92%, 93%, 94%, 95%, 96%, 97%, 98% or 99% identity to the mature polypeptide in the amino acid sequence of SEQ ID NO: 2 and that exhibits at least one activity of the mature polypeptide in the amino acid sequence according to SEQ ID NO: 2.
33. The use according to embodiments 30-32, wherein the isolated polypeptide being at least 70% identical to the mature polypeptide in SEQ ID NO: 2 is the mature polypeptide in SEQ ID NO: 4, 6 or 8.
34. The use according to embodiment 30-33, wherein the mature polypeptide in SEQ ID NO: 2, 4, 6 or 8 is amino acids 34 to 304 in the amino acid sequence according to SEQ ID NO: 2, 4, 6, or 8.
35. The use according to embodiments 30-34, wherein the cake is an egg and/or fat reduced cake.
36. The use according to embodiments 30-35, wherein the cake is an emulsifier-free cake.
37. Use of an anti staling amylase, preferably a maltogenic α-amylase, more preferably an isolated polypeptide according to SEQ ID NO: 9 or an isolated polypeptide being at least 70% identical thereto to improve at least one cake property selected from: (i) initial crumb softness, (ii) crumb softness upon storage, (iii) shelf life, (iv) cohesiveness and/or (v) cake volume.
38. Use according to embodiment 37 wherein the anti staling amylase is a maltogenic α-amylase, preferably an isolated polypeptide according to SEQ ID NO: 9.
39. Use according to embodiment 37-38, wherein the anti staling amylase is an isolated polypeptide at least 70% to SEQ ID NO:9, preferably at least 75%, 80%, 85%, 90%, 91%, 92%, 93, 94%, 95%, 96%, 97%, 98% or 99% identical thereto.
40. Use according to embodiment 39 wherein the isolated polypeptide being at least 70% identical to SEQ ID NO: 9 has the ability to form cyclodextrin when acting on starch and is a maltogenic α-amylase variant, preferably the maltogenic α-amylase variant includes, if compared with SEQ ID NO: 9, a deletion in the region 190-195 in SEQ ID NO: 9, preferably a deletion of amino acid 191 to 195 and/or a substitution of amino acid 188 and/or 189.
41. Use according to embodiment 40, wherein the isolated polypeptide having anti staling amylase activity being at least 70% identical to SEQ ID NO: 9, preferably being at least 75%, 80%, 85%, at least 90%, 91%, 92%, 93%, 94%, 95%, 96%, 97%, 98% or 99% identical thereto comprises an amino acid modification compared to SEQ ID NO: 9 at a position that corresponds to residue F188, preferably corresponds to residue F188I or F188L.
42. Use according to embodiment 39-41 wherein the isolated polypeptide being at least 70% identical to SEQ ID NO: 9 comprises a modification at one or more positions corresponding to the amino acid residues in SEQ ID NO: 9: A30, K40, N115, T142, F188, T189, P191, A192, G193, F194, S195, D261, N327, K425, K520 and N595, more preferably the variant comprises one or more modifications corresponding to the amino acids in SEQ ID NO: 9: A30D, K40R, N115D, T142A, F188L, T189Y, Δ (191-195), D261 G, N327S, K425E, K520R and N595I.
43. Use according to embodiment 39-42 wherein the isolated polypeptide being at least 70% identical to SEQ ID NO: 9 has an amino acid sequence which compared with SEQ ID NO: 9 has one or more amino acid alterations which is a substitution, a deletion or an insertion at one of the a position I15, R18, K44, N86, T87, G88, Y89, H90, Y92, W93, F188, T189, D190, P191 , A192, F194, L196, L225, F252, D261, T288, I290 D329, N371 , D372, P373, N375 or R376, S446, G469, S578 or P642, preferably a substitution, a deletion or an insertion at one of the a position N86T/VG/K, T87N/Q, H90W/F/Y/R/M, F188I/L/T/G/V, D190G, P191*, A192G/Q/R, F194S/L/Y, L196F, L225S, F252L, D261G, T288P, I290V, N371 K/R/F/Y/Q, D372E/Q/S/T/A/V, N375S, S446A, G469R, S578G or P642Q.
44. Use according to embodiment 43 wherein the isolated polypeptide having at least 70% identity to SEQ ID NO: 9 has an amino acid sequence which compared with SEQ ID NO: 9 has the following combinations of amino acid alterations:
   D261G + T288P;
   F188L + D261G + T288P;
   T288P;
   Y89F + D261G + T288P;
   N86V + F188L + D261G + T288P;
   Y89F + F188L + D261G + T288P;
   Y89H + F188L + D261G + T288P;
   N86T + F188L + D261G + T288P;
   F194S + D261G + T288P;
   L196F;
   D261G + T288P + D372V;
   Q184H + N187D + F194Y;
   D190G;
   N86G + Y89M + F188L + D261G + T288P;
   F188L + D190G + D261G + T288P;
   A192Q + D261G + T288P + S446A;
   F188H;
   P191*;
   A192*;
   A192* + G193*;
   *192aA;
   N86K + F252L + D261 G + T288P;
   F194Y + L225S + 261 G + T288P;
   F194L + D261G + T288P;
   F194S+ D261G + T288P + P642Q;
   D261G + T288P + N375S;
   F188T;
   F188G;
   F188V;
   A192R + F194L + D261G + T288P + G469R;
   A192G + D261 G + T288P;
   Y89F + D261G + T288P + I290V + N375S.
45. Use according to embodiment 44 wherein the isolated polypeptide having anti staling amylase activity being at least 70% identical, preferably being at least 75%, 80%, 85%, at least 90%, 91%, 92%, 93%, 94%, 95%, 96%, 97%, 98% or 99% identical thereto, comprises the combination of amino acid alterations F188L+D261G+T288P compared to SEQ ID NO: 9.
46. Use according to embodiment 43-45 wherein the isolated polypeptide having at least 70% identity to SEQ ID NO: 9, comprises the two substitutions D261G and T288P and at least one additional amino acid alteration which is a substitution, a deletion or an insertion at position Y89, W93, P191, F194, Y360 or N375.
47. Use according to embodiment 46 wherein the isolated polypeptide having at least 70% identity to SEQ ID NO: 9 has an amino acid sequence which compared with SEQ ID NO: 9 has one of the following sets of alterations:
   F194Y + D261G + T288P + N375S;
   I15T + P191S + D261G + T288P + N375S + S640I;
   Y89F + P191S + D261G + T288P;
   I15T + Y89F + P191S + D261G + T288P;
   Y89F + F194Y + D261G + T288P;
   Y89F + D261G + T288P + N375S;
   Y89F + P191S + F194Y + D261G + T288P;
   Y89F + P191S + D261G + T288P + N375S;
   Y89F + P191S + D261G + T288P + Y360N;
   Y89F + P191S + D261G + T288P + Y360F;
   Y89F + W93Y + P191S + D261G + T288P;
   Y89F + W93F + P191S + D261G + T288P;
   Y89F + P191S + F194Y + D261G + T288P + N375S.

### Examples

### Materials and Methods

### Enzyme activity assays

### Lipase activity (DLU)

### Unit definition

One DLU is defined as the amount of enzyme that liberates 1 micromol p-nitrophenol per minute under the conditions of the test (pH 8.5, 37°C).

### Assay

The lipolytic activity was determined in an assay with the chromogenic substrate p-nitrophenyl palmitate (pNPP). The substrate (Sigma N2752) was dissolved in 2-propanol (3 mg/mL). While vigorously stirring 3.5 mL of this solution was drop wise added to 46.5 mL 100 millimol/I TRIS buffer pH 8.5 containing 1% Triton X-100. At time t=0, 50 µL of sample was mixed with 1 mL substrate solution. While incubating at 37 °C the change in absorption was measured at 405 nm against a sample blank. The slope (deltaOD/time) of the linear part of the curve is used as measure for the activity. The activity is expressed in DLU (DSM Lipase Units).

### Phospholipase activity (APLU)

### Unit definition

One phospholipase A unit (APLU) is defined as the amount of enzyme that liberates 1 micromole of 4-thiopyridone per minute at the reaction conditions stated (0.1 M acetate buffer pH 4.0 + 0.2% Triton-X100 at 37°C).

### Assay

Phospholipase A activity is determined spectrophotometrically by using 1,2-dimercaptodioctanoyl-phosphatidylcholine as a substrate. Phospholipase A hydrolyses the thioester bond at the 1 position (PLA1) or at the 2 position (PLA2) thereby liberating octamoic acid and 1,2-dimercapto-mono-octanoyl-phosphatidylcholine or 1,2-dimercapto-phosphatidylcholine. The liberated thiol groups are titrated in a subsequent reaction with 4,4'-dithiopyridine to form 4-thiopyridone. The latter is in tautomeric equilibrium with 4-mercaptopyridine that absorbs at 334 nm. The reaction is carried out in 0.1 M acetate buffer pH 4.0 + 0.2% Triton-X100 at 37°C.

### Galactolipase activity

### Unit definition

One galactolipase unit is defined as the amount of enzyme that forms 1 micromole of fatty acid per minute at the reaction conditions stated (50 mM acetic acid buffer, 4 mM CaCl2, 0.2% Triton X-100 and 1 mg/ml digalactosyldiglyceride (Lipid Products), pH 4.5, 30 minutes at 30°C).

### Assay

Galactolipase activity is determined with H-NMR spectroscopy by using digalactosyldiglyceride as a substrate, according to the method described by Hirayama and Matsuda (1972) Agric. Biol. Chem. 36, 1831. Galactolipase hydrolyses the ester bond between the fatty acids and the glycerol backbone thereby liberating one or both fatty acids. The reaction is carried out in 50 mM acetic acid buffer pH 4.5 further containing 4 mM CaCl2, 0.2% Triton X-100 and 1 mg/ml digalactosyldiglyceride (Lipid Products) for 30 minutes at 30°C.

### Maltogenic α-amylase activity (MANU)

The activity of the maltogenic α-amylase may be determined using the MANU method as an assay (Food Chemical Codex 5th edition, page 916-917).

One MANU is defined as the amount of enzyme required to release 1 µmol of maltose per minute at a concentration of maltotriose substrate of 10 mg/ml in 0.1M citrate buffer at pH 5.0, 37°C and for 30 minutes.

### Phospholipase A2 activity (EYU)

The activity of phospholipase A2 is determined according to the Egg Yolk Unit assay described in the Food Chemical Codex, 5th Edition, page 920-921.

One EYU (egg yolk unit, indicated as "phospholipase unit" in the Food Chemical Codex 5th edition) is defined as the amount of enzyme producing one micromole (µmole) of free fatty acid per minute under the conditions described for an egg yolk assay in the Food Chemicals Codex, 5th Edition, page 920-921.

### Bacterial amylase activity (RAU)

### Unit definition

1 RAU is defined as the amount of enzyme that convert under standardized conditions (pH=6.6, 30°C) 1 mg soluble starch per minute, having an equal absorption to a reference colour at 620 nm after reaction with Iodine

### Principle of enzyme assay

Bacterial amylase hydrolyses starch into dextrines and maltose. The mixture is coloured by the addition of iodine. The absorbance is measured spectrophotometrically at 620 nm. The time needed for the incubation mixture to reach the same absorbance as that of a colour reference (CoCl2 solution) is a measure for the enzyme activity.

### Assay

Enzyme solution is mixed with starch substrate (soluble starch 13.3 mg/mL) and incubated at 30°C and pH 6.6 in phosphate buffer. After regular time intervals a sample is mixed with iodine solution and the absorption is measured at 620 nm. Sampling will proceed until the absorption is less than the absorption of the color standard. The log (absorbance) against the time is plotted. Using linear regression, the time needed to reach the absorption of the color standard is calculated and used as measure for the activity.

1 RAU unit corresponds to 1 BAU unit. The BAU assay and BAU unit is described in the Food Chemical Codex 6th Edition, page 1114-1115.

### Cake recipes

Four basic plain cake recipes were used in the examples. Shelf life of the cake produced with the enclosed recipes was of at least eight weeks. Standard, plain cake recipes have a shelf life of about six weeks at ambient temperature. To prolong this shelf life pH was decreased with citric acid and the water activity was decreased by replacing some sugar for glycerol. Also potassium sorbate was added to inhibit mould grow. Mould growth was not encountered in practice by following this approach.

### Pound cakes

The first column shows the plain pound cake, second column shows cake without emulsifier. No further adjustments have been made for this recipe. And the last column shows the recipe with 80% egg. The 20% egg reduction was replaced with 100% water to maintain mass/moisture balance.

**Table 1. Recipes pound cake, pound cake without emulsifier and pound cake with reduced egg**

| | **Plain pound cake** | **Pound cake with no emulsifier** | **Pound cake with reduced egg (-20%)** |
|---|---|---|---|
| **Ingredient** | **%** | **%** | **%** |
| Whole Egg (pasteurised) | 24.4 | 24.5 | 19.5 |
| Flour¹ | 24.4 | 24.5 | 24.4 |
| Sugar (caster) | 23.4 | 23.5 | 23.4 |
| Butter | 24.4 | 24.5 | 24.4 |
| Glycerol | 2.00 | 2.04 | 2.04 |
| Paste emulsifier² | 0.20 | - | 0.20 |
| Sodium bicarbonate | 0.20 | 0.20 | 0.20 |
| SAPP 28 | 0.33 | 0.33 | 0.33 |
| Salt | 0.24 | 0.24 | 0.24 |
| Potassium sorbate | 0.16 | 0.16 | 0.16 |
| Citric acid | 0.14 | 0.14 | 0.14 |
| Water | - | - | 4.9 |

| | | | |
|---|---|---|---|
| ¹ Albatros (heat-treated wheat flour, Maneba, The Netherlands) ² Ovalett (Bakels, The Netherlands) | | | |

The dosages of enzymes in the examples are based on total cake batter weight).

The pH of this cake was 6.5 - 6.7, with a water activity of 0.83- 0.85

### Mixing conditions plain pound cake en pound cake without emulsifier:

The butter, flour and emulsifier where creamed together using a planetary mixer (Hobart, 12 L) in 2 min (speed 1=low speed). Add other dry (and sieved) ingredients were blended for 5 min (speed 2=medium speed) and the egg were added in 3 steps, speed 1 for 2 min.

After mixing, 8 tins per recipe, sprayed with Goldwax (Sonneveld) as a releasing agent, were filled with 400 g batter each.

### Baking conditions plain pound cake en pound cake without emulsifier:

The plain pound cakes were baked using the following conditions: Oven temperature: 155°C top heat, 150°C bottom heat for 60 min

The cakes without emulsifier were baked using the following conditions: Oven temperature: 160°C top heat, 150°C bottom heat, 60 min

### Mixing conditions pound cake with reduced eggs

Use a planetary mixer (Hobart, 12 L) and a paddle.

The butter was mixed in the Hobart mixer for 3 min. (speed 2). Subsequently all other ingredients were added and mixed for 6 min. (speed 2).

After mixing, 7 tins per recipe, sprayed with Goldwax (Sonneveld) as a releasing agent, were filled with 400 g batter each.

### Baking conditions pound cake with reduced egg:

These cakes were baked using the following conditions: Oven temperature: 155°C top heat, 150°C bottom heat for 67 min..

After baking, the cakes were immediately released from the tin. After cooling (temp<25°C) the cakes were packed, in thick foil (35 µm, PPO) and sealed to prevent moisture loss. Cakes were stored in dark at an ambient temperature (18-22°C).

### High ratio cakes

High ratio cakes have a higher amount of sugar and/or water than flour. This makes the cakes much sweeter in taste and it has a smoother/softer texture than standard pound cake.

**Table 2 Recipe High Ratio cake**

| **Ingredients** | **%** |
|---|---|
| Egg (pasteurized) | 18.5 |
| Flour¹ | 23.1 |
| Sugar (caster) | 26.6 |
| Cream margarine (80% fat) | 13.4 |
| Water | 12.7 |
| Skimmed milk powder | 1.6 |
| Glycerol | 2.3 |
| Paste emulsifier² | 0.46 |
| Sodium bicarbonate | 0.36 |
| SAPP 28 | 0.50 |
| Salt | 0.23 |
| Potassium sorbate | 0.17 |
| Citric acid | 0.14 |
| Total | 100 % |

| | |
|---|---|
| ¹ Flamingo (Heat treated wheat flour, Maneba, The Netherlands) ² Ovalett (Bakels, The Netherlands) | |

The pH of the high ratio cake is 6.8 - 7.0, with a water activity of 0.83- 0.85.

### Mixing conditions:

Butter and emulsifier were creamed together in an Hobart mixer (2 min, second speed). The remaining ingredients (sieved and pre-blended) were added and mixed together for 1 min, first speed. Finally the batter was mixed for 5 min in third speed (=high speed). The density of the high ratio cake should be about 0.78 kg/L.

After mixing 7 cake tins, sprayed with Goldwax (Sonneveld), were filled with 400 g batter each.

### Baking conditions:

These cakes were baked under the following conditions: oven temperature: 165°C top heat, 175°C bottom heat for 50 min..

After baking and cooling (temp<25°C) the cakes were packed in thick foil (35 µm, PPO) and sealed. The cakes were stored in the dark at ambient temperature (18-24°C).

### Muffins

A muffin recipe contains oil instead of fat. A good muffin has a high specific volume and big crevices. No citric acid is added in this recipe as the expected shelf life was already acceptable. This is mainly due to the rather low water activity of a muffin (about 0.78).

**Table 1 Muffins recipe**

| **Ingredient** | **%** |
|---|---|
| Egg (pasteurized) | 14.4 |
| Flour¹ | 25.9 |
| Sugar (caster) | 24.0 |
| Oil (sunflower) | 19.2 |
| Water | 8.6 |
| Skimmed milk powder | 2.9 |
| Glycerol | 2.9 |
| GMS² | 0.48 |
| Sodium bicarbonate | 0.38 |
| SAPP 28 | 0.61 |
| Salt | 0.29 |
| Potassium sorbate | 0.16 |
| Xanthan gum | 0.14 |
| Total | 100 % |

| | |
|---|---|
| ¹ Ibis (Protein-reach wheat flour, Maneba, The Netherlands) ² Nutisoft 55AC (Cognis Germany) | |

The pH of the muffins is 7 - 7.5, with a water activity of 0.76- 0.78

### Mixing conditions:

The dry ingredients were blended in a Hobart after which the liquid ingredients were added and mixed at first speed for 1 minute, then 2 min. in second speed.

After mixing muffin trays were filled with 65 g batter each. Baking conditions muffins:

### Baking conditions

These cakes were baked at oven temperature: 230°C top heat and 190°C bottom heat for 24 min.

After baking and cooling (temp<25°C) the cakes were packed in thick foil (35 µm, PPO) and sealed. The cakes were stored in the dark at ambient temperature (18-24°C).

### Sponge cake recipe

A lot of different recipes for sponge could be used, which differ mainly in type of flour and presence of oil. In the examples a plain sponge recipe without fat (West European style) was used.

**Table 4 Recipe sponge cake**

| **Ingredient** | **%** |
|---|---|
| Egg (pasteurized) | 29.6 |
| Flour¹ | 20.5 |
| Sugar (caster) | 28.4 |
| Wheat starch | 8.0 |
| Water | 9.1 |
| sponge emulsifier (BV 40) | 3.2 |
| Sodium bicarbonate | 0.41 |
| SAPP 28 | 0.57 |
| Potassium sorbate | 0.14 |
| Citric acid | 0.11 |
| Total | 100% |

| | |
|---|---|
| ¹ Albatros (heat-treated wheat flour, Meneba, The Netherlands) | |

### Mixing conditions:

All ingredients were mixed in a Hobart mixer for 10 min in the 3rd speed, and finally 1 min. in first speed.

After mixing, a plate (60 x 40 cm) was filled with 2.0 kg batter.

### Baking conditions:

These cakes were baked in the oven at a temperature: 180°C top temperature, 180°C bottom temperature for 28 min.

After baking and cooling (temp<25°C) the sponge cakes were sliced. On all sides the outer layer (5 cm) was cut off. Pieces of 10 x 30 cm were packed in thick foil (35 µm, PPO) and sealed. The cakes were stored in the dark at ambient temperature (18-24°C).

The shelf life of sponges is not as long as pound cakes and so cakes were stored for just 5 weeks.

### Example 1

### Effect of a baking composition according to the invention comprising lipolytic enzyme and an anti staling amylase on the softness and volume of a pound cake

Plain pound cakes were produced according to the recipe in this specification.

In this example the lipolytic enzyme according to amino acid 34-304 in SEQ ID NO: 2 and the maltogenic α-amylase according to SEQ ID NO; 9 were used separately or as a baking composition in the production of pound cakes. The initial crumb hardness of the cake was measured after 24 hours, while the crumb hardness upon storage was measured after 2, 6 and 12 weeks. The crumb softness corresponds to the opposite of the crumb hardness. The amount of enzymes used is reported in tables 5a to 5c as well as the corresponding relative volume of the cakes and the crumb hardness measured at different times.

The lipolytic enzyme according to amino acid 34-304 in SEQ ID NO: 2 is sold under the trade mark Panamore^{™} Golden (DSM Food Specialties, The Netherlands). The maltogenic α-amylase according to SEQ ID NO: 9 is sold under the trade mark Bakezyme™ MA 10.000 (DSM Food Specialties, The Netherlands) or under the trade mark Novamyl® (Novozymes, Denmark).

In this experiment enzyme preparations were used comprising the following enzyme units/g:
Panamore™ Golden: 2750 DLU/g (indicated as Panamore in the tables)
Novamyl®: 11000 MANU/g

**Table 5a. Pound Cake: crumb hardness during storage (compression force [g]); effect of Panamore™ Golden**

| **storage time (weeks)** | **No enzyme** | **Panamore, 275 DLU*** | **Panamore, 390 DLU*** | **Panamore, 500 DLU*** |
|---|---|---|---|---|
| | | | | |
| 0 | 1743 | 1458 | 1453 | 1515 |
| 2 | 3127 | 2150 | 2013 | 2019 |
| 6 | 3546 | 2351 | 2197 | 2228 |
| 12 | 3698 | 2517 | 2360 | 2383 |
| | | | | |
| relative volume (%) | 100 | 104 | 104 | 102 |

**Table 5b. Pound Cake: crumb hardness during storage (compression force [g]); effect of Novamyl®**

| **storage time (weeks)** | **No enzyme** | **Novamyl, 1600 MANU*** | **Novamyl, 2200 MANU*** | **Novamyl, 2800 MANU*** |
|---|---|---|---|---|
| | | | | |
| 0 | 1743 | 1715 | 1763 | 1846 |
| 2 | 3127 | 2958 | 2961 | 3004 |
| 6 | 3546 | 3296 | 3291 | 3340 |
| 12 | 3698 | 3194 | 3117 | 3113 |
| | | | | |
| relative volume (%) | 100 | 102 | 101 | 99,8 |

**Table 5c. Pound Cake: crumb hardness during storage (compression force [g]); effect of Panamore™ Golden and Novamyl® in combination**

| **storage time (weeks)** | **No enzyme** | **Panamore, 275 DLU* Novamyl, 1600 MANU*** | **Panamore, 390 DLU* Novamyl, 2200 MANU*** | **Panamore, 500 DLU* Novamyl, 2800 MANU*** |
|---|---|---|---|---|
| | | | | |
| 0 | 1743 | 1356 | 1379 | 1379 |
| 2 | 3127 | 1981 | 1897 | 1897 |
| 6 | 3546 | 2102 | 2022 | 2022 |
| 12 | 3698 | 2141 | 2212 | 2212 |
| | | | | |
| relative volume (%) | 100 | 106 | 105 | 102 |

| | | | | |
|---|---|---|---|---|
| * The amount of enzymes in table 5a to 5c refers to 1 kg batter. | | | | |

From Table 5a it is clear that the addition of the lipolytic enzyme Panamore™ Golden has a very good effect on cake volume of plain pound cake if compared with that of a pound cake made without addition of enzyme. The crumb softness, both initial and upon storage, is also considerably improved. It is noticed that very good effect are already obtained at 275DLU/kg batter and increasing the enzyme dosage to 390 or 500 DLU/kg batter does not lead to a significant improvement in the cake softness. The addition of the enzyme Novamyl® to the cake (Table 5b) does not have significant effect on the cake volume and on the initial cake softness; the improvement is more evident on the cake softness upon storage. Changing the dosage from 1600 MANU to 2800 MANU has no effect on the cake softness and volume.

The addition of baking enzyme compositions comprising both Panamore™ Golden and Novamyl® (Table 5c) has an extraordinary effect on both cake volume and on cake softness initial and upon storage, which are even further improved if compared with the cake comprising Panamore™ alone. This experiment shows that a baking composition according to the invention when added to plain pound cake, can considerably improve the volume of the cake. Furthermore firmness of the cake crumb is considerably delayed leading to improved shelf life of the cake.

### Example 2

### Effect of a baking composition according to the invention comprising lipolytic enzyme and an anti staling amylase on the softness and volume of muffins

Muffins were produced according to the recipe in this specification.

Also in this example the effect of the lipolytic enzyme Panamore™ Golden, of the antistaling amylase Novamyl® and of a baking composition comprising both enzymes was evaluated. The initial crumb hardness of the cake was measured after 24 hours, while the crumb hardness upon storage was measured after 2 and 8 weeks. The amount of enzymes used is reported in tables 6a to 6c as well as the corresponding relative volume of the cakes and the crumb hardness measured at different times. The same enzyme preparations were used as in Example 1.

**Table 6a. Muffin: crumb hardness during storage (compression force [g]); effect of Panamore™ Golden**

| **storage time (weeks)** | **No enzyme** | **Panamore, 400 DLU*** | **Panamore, 470 DLU*** | **Panamore, 540 DLU*** |
|---|---|---|---|---|
| | | | | |
| 0 | 873 | 611 | 583 | 563 |
| 2 | 1060 | 719 | 700 | 696 |
| 8 | 1251 | 914 | 913 | 935 |
| | | | | |

**Table 6b. Muffin: crumb hardness during storage (compression force [g]); effect of Novamyl®**

| **storage time (weeks)** | **No enzyme** | **Novamyl, 3600 MANU*** | **Novamyl, 4200 MANU*** | **Novamyl, 4800 MANU*** |
|---|---|---|---|---|
| | | | | |
| 0 | 873 | 837 | 830 | 824 |
| 2 | 1060 | 1062 | 1062 | 1063 |
| 8 | 1251 | 1139 | 1119 | 1100 |
| | | | | |

**Table 6c. Muffin: crumb hardness during storage (compression force [g]); effect of Panamore™ Golden and Novamyl® in combination**

| **storage time (weeks)** | **No enzyme** | **Panamore, 400 DLU* Novamyl, 3600 MANU*** | **Panamore, 470 DLU* Novamyl, 4200 MANU*** | **Panamore, 540 DLU* Novamyl, 4800 MANU*** |
|---|---|---|---|---|
| | | | | |
| 0 | 873 | 575 | 541 | 515 |
| 2 | 1060 | 721 | 702 | 699 |
| 8 | 1251 | 801 | 781 | 783 |
| | | | | |

| | | | | |
|---|---|---|---|---|
| * The amount of enzymes in table 6a to 6c refers to 1 kg batter. | | | | |

From Table 6a it is clear that the addition of the lipolytic enzyme Panamore™ Golden has a very good effect on the crumb softness, both initial and upon storage of muffins if compared with muffins not comprising the enzyme. It is noticed that very good effect are already obtained at 400DLU/kg batter and increasing the enzyme dosage to 470 or 540 DLU/kg batter does not lead to a significant further improvement in the cake softness upon storage.

The addition of the enzyme Novamyl® to muffins (Table 6a) does not have significant effect on the cake volume and on the initial crumb softness and on the crumb softness 2 weeks upon storage. The improvement is only evident on the cake softness upon 8 week storage. No effect of enzyme dosage increase is observed.

The addition of baking enzyme compositions comprising both Panamore™ Golden and Novamyl® (Table 6 c) has a good effect on cake softness initial and upon storage, and is even further improved if compared with the muffins comprising Panamore™ alone. This experiment shows that a baking composition according to the invention when added to plain pound cake can retard firmness of the muffin crumb leading to improved shelf life of the muffin.

### Example 3

### Effect of a baking composition according to the invention comprising lipolytic enzyme, an anti staling amylase and optionally a phospholipase A2 and a bacterial amylase, on the softness and volume of high ratio cake

High ratio cakes were produced according to the recipe in this specification.

In this example the effect of the lipolytic enzyme Panamore™ Golden, of the antistaling amylase Novamyl® and of a baking composition comprising both enzymes was evaluated. The initial crumb hardness of the cake was measured after 24 hours, while the crumb hardness upon storage was measured after 2, 4 and 12 weeks. The amount of enzymes used is reported in tables 7a to 7c as well as the corresponding relative volume of the cakes and the crumb hardness measured at different times. The same enzyme preparations for Panamore™ Golden and Novamyl® were used as in Example 1.

In Table 7d the effect on volume and crumb softness of a baking composition comprising the lipolytic enzyme, the anti staling amylase, a phospholipase A2 and a bacterial amylase is shown.

The phospholipase A2 is the enzyme sold under the trade mark Cakezyme™ (DSM Food Specialties, The Netherlands) while the bacterial amylase is a bacterial α-amylase from *Bacillus amyloliquefaciens* sold under the trade mark BAN™ (Novozymes, Denmark). The enzyme preparation used have the following enzyme units/g:
Cakezyme™: 5000 EYU/g
BAN™: 50000 RAU/g.

**Table 7a. High Ratio Cake: crumb hardness during storage (compression force [g]); effect of Panamore™ Golden**

| **storage time (weeks)** | **No enzyme** | **Panamore, 40 DLU*** | **Panamore, 70 DLU*** | **Panamore, 100 DLU*** |
|---|---|---|---|---|
| | | | | |
| 0 | 1235 | 1128 | 1089 | 1240 |
| 2 | 2084 | 1645 | 1693 | 1695 |
| 4 | 2266 | 1776 | 1754 | 1982 |
| 12 | 2764 | 2009 | 2103 | 2074 |
| | | | | |
| Relative volume (%) | 100 | 106 | 104 | 107 |

**Table 7b. High ratio Cake: crumb hardness during storage (compression force [g]); effect of Novamyl®**

| **storage time (weeks)** | **No enzyme** | **Novamyl, 1900 MANU*** | **Novamyl, 2550 MANU*** | **Novamyl, 3200 MANU*** |
|---|---|---|---|---|
| | | | | |
| 0 | 1235 | 1160 | 1174 | 1206 |
| 2 | 2084 | 1809 | 1786 | 1797 |
| 4 | 2266 | 2138 | 2167 | 2232 |
| 12 | 2764 | 2625 | 2619 | 2634 |
| | | | | |
| relative volume (%) | 100 | 106 | 107 | 106 |

**Table 7c. High Ratio Cake: crumb hardness during storage (compression force [g]); effect of Panamore™ Golden and Novamyl® in combination**

| **storage time (weeks)** | **No enzyme** | **Panamore, 40 DLU* Novamyl, 1900 MANU*** | **Panamore, 70 DLU* Novamyl, 2550 MANU*** | **Panamore, 100 DLU* Novamyl, 3200 MANU*** |
|---|---|---|---|---|
| | | | | |
| 0 | 1235 | 1015 | 1067 | 1211 |
| 2 | 2084 | 1576 | 1663 | 1935 |
| 4 | 2266 | 1626 | 1677 | 1949 |
| 12 | 2764 | 1963 | 1864 | 1943 |
| | | | | |
| Relative volume (%) | 100 | 110 | 112 | 113 |

**Table 7d. High Ratio Cake: crumb hardness during storage (compression force [g]); effect of Panamore™ Golden, Novamyl®, Cakezyme™ and BAN™ in combination**

| **storage time (weeks)** | **No enzyme** | **BAN, 20 RAU*** | **Cakezyme, 550 EYU*** | **Panamore, 100 DLU*** | **Panamore, 100 DLU*** |
|---|---|---|---|---|---|
| | | | | **Novamyl, 3200 MANU*** | **Novamyl, 3200 MANU* BAN, 20 RAU* Cakezyme, 550 EYU*** |
| | | | | | |
| 0 | 1235 | 1223 | 1240 | 1211 | 1061 |
| 2 | 2084 | 2114 | 2050 | 1935 | 1529 |
| 4 | 2266 | 2215 | 2240 | 1949 | 1601 |
| 12 | 2764 | 2601 | 2682 | 1943 | 1619 |
| | | | | | |
| relative volume (%) | 100 | 99 | 104 | 113 | 115 |

| | | | | | |
|---|---|---|---|---|---|
| * The amount of enzymes in table 7a to 7d refers to 1 kg batter. | | | | | |

Table 7a shows that the addition of Panamore™ Golden in the production of high ratio cake considerably improved the cake volume. The initial crumb softness was only improved at low enzyme dosages. The crumb softness upon storage was considerably improved but no effect of enzyme dosage was observed.

From Table 7b it can be seen that the addition of Novamyl® in the production of high ratio cake also lead to considerable improvement of cake volume. The initial crumb softness and the crumb softness upon storage were improved but higher enzyme dosages lead to slight or almost no effect.

From Table 7c it can be seen that compositions both comprising Panamore™ Golden and Novamyl® had excellent effect on both volume and improvement of initial crumb softness and the crumb softness upon storage.

Addition to the baking composition of further enzymes, i.e. BAN™ and Cakezyme™ lead to even further improvement of cake volume and both initial crumb sofness and crumb softness upon storage.

This experiment shows that a baking composition according to the invention further comprising a phospholipase and a bacterial α-amylase when added to high ratio cake can retard firmness of the cake crumb leading to improved shelf life of the cake and can considerably improve the cake volume.

## Claims

1. Method to produce a cake comprising:
a) preparing a cake batter starting from cake batter ingredients,
b) baking the cake batter to yield the cake,
wherein in the preparation of the cake batter a baking composition comprising a lipolytic enzyme and an anti staling amylase or a cake pre-mix comprising flour and a baking composition comprising a lipolytic enzyme and an anti staling amylase is added in an effective amount,
wherein the lipolytic enzyme is an isolated polypeptide having the amino acid sequence according to the mature polypeptide sequence in SEQ ID NO: 2 or an isolated polypeptide being at least 70% identical to said mature polypeptide sequence,
wherein the anti staling amylase is an isolated polypeptide according to SEQ ID NO: 9 or an isolated polypeptide being at least 70% identical thereto.

2. Use of a baking composition comprising a lipolytic enzyme and an anti staling amylase or of a cake pre-mix comprising flour and a baking composition comprising a lipolytic enzyme and an anti staling amylase in the production of cake to improve one or more batter or cake properties, and/or to allow the reduction of eggs and/or fat in the cake, wherein the lipolytic enzyme is an isolated polypeptide having the amino acid sequence according to the mature polypeptide sequence in SEQ ID NO: 2 or an isolated polypeptide being at least 70% identical to said mature polypeptide sequence and wherein the anti staling amylase is an isolated polypeptide according to SEQ ID NO: 9 or an isolated polypeptide being at least 70% identical thereto.

3. Method according to claim 1, or use according to claims 2 wherein the lipolytic enzyme is selected from the polypeptide having the amino acid sequence according to the mature polypeptide sequence in SEQ ID NO: 2, 4, 6 or 8.

4. Method according to claims 1-3 or use according to claims 2-3 wherein the anti staling amylase is a maltogenic α-amylase according to SEQ ID NO: 9.

5. Method according to claims 1-4 or use according to claims 2-4 wherein the anti staling amylase is a polypeptide having at least 70% identity to SEQ ID NO: 9 and has an amino acid sequence which compared with SEQ ID NO: 9 has one or more amino acid alterations which is a substitution, a deletion or an insertion at position I15, R18, K44, N86, T87, G88, Y89, H90, Y92, W93, F188, T189, D190, P191 , A192, F194, L196, D329, N371 , D372, P373, N375 or R376 preferably a substitution, a deletion or an insertion at one of the a position N86T/VG/K, T87N/Q, H90W/F/Y/R/M, F1881/L/T/G/V, D190G, a deletion at position P191, A192G/Q/R, F194S/L/Y, L196F, L225S, F252L, D261 G, T288P, I290V, N371 K/R/F/Y/Q, D372E/Q/S/T/A/V, N375S, S446A, G469R, S578G or P642Q.

6. Method or use according to claim 5 wherein the anti-staling α-amylase is a polypeptide having at least 70% identity to SEQ ID NO: 9, comprising the two substitutions D261 G and T288P and at least one additional amino acid alteration which is compared to SEQ ID NO: 9 a substitution, a deletion or an insertion at position Y89, W93, P191, F194, Y360 or N375.

7. Method according to claims 1-6 or use according to claims 2-6 wherein the baking composition further comprises other enzymes, preferably further comprises one or more enzymes selected from a phospholipase, and a bacterial α-amylase or a combination thereof.

8. Method according to claim 1-7 or use according to claim 2-7 wherein the baking composition or a cake pre-mix comprising flour and the baking composition can be used to improve at least one property selected from (i) initial crumb softness, (ii) crumb pore homogeneity, (iii) crumb pore diameter, (iv) crumb softness upon storage, (v) shelf life, (vi) cohesiveness and/or (vii) cake volume in the cake.

9. Method according to claim 1-8 or use according to claim 2-8 wherein the baking composition or a cake pre-mix comprising flour and the baking composition can be used to improve at least one of the properties selected from (i) batter viscosity, (ii) specific density in the cake batter.

10. Method according to claim 1-9 or use according to claim 2-9 wherein the baking composition or a cake pre-mix comprising flour and the baking composition can be used to reduce the amount of eggs and/or fat used in the recipe whilst at least maintaining at least one of the properties selected from the group consisting of: (i) batter viscosity, (ii) specific density, (iii) initial crumb softness, (iv) crumb pore homogeneity, (v) crumb pore diameter, (vi) crumb softness upon storage, (vii) shelf life, (viii) cohesiveness and/or (ix) cake volume.

11. Method according to claim 1-10 or use according to claims 2-10 wherein the lipolytic enzyme is added in an amount of 10 - 2000 DLU/kg of cake batter, preferably in an amount of 50 - 600DLU/kg of cake batter.

12. Method according to claims 1-11 or use according to claims 2-11 wherein the anti staling amylase is added in an amount of 250 -6000 MANU/kg of cake batter, preferably in an amount of 500 - 3000 MANU /kg of cake batter.

13. Method according to claims 1-12 or use according to claims 2-12 wherein the cake comprises a reduced amount of eggs and/or fat if compared with a conventional cake.

14. Method or use according to claim 13 wherein the amount of eggs is reduced with at least 5% w/w, preferably with at least 10% w/w, more preferably with at least 15% w/w, most preferably the amount of eggs is reduced with at least 20% w/w, 30% w/w, 40% w/w, 50% w/w.

15. Method or use according to claim 13-14 wherein the amount of fat is reduced with at least 10% w/w, preferably the amount of fat is reduced with at least 20% w/w, even more preferably the amount of fat is reduced with at least 30% w/w.

16. Use of a lipolytic enzyme which is an isolated polypeptide having the amino acid sequence according to the mature polypeptide sequence in SEQ ID NO: 2 or an isolated polypeptide being at least 70% identical to said mature polypeptide sequence to improve at least one cake property selected from: (i) volume, (ii) initial crumb softness, (iii) crumb softness upon storage and/or (iv) cohesiveness.

17. The use according to claim 16, wherein the isolated polypeptide being at least 70% identical to the mature polypeptide in SEQ ID NO: 2 is the mature polypeptide in SEQ ID NO: 4, 6 or 8.

18. The use according to claim 16-17, wherein the mature polypeptide in SEQ ID NO: 2, 4, 6 or 8 is amino acids 34 to 304 in the amino acid sequence according to SEQ ID NO: 2, 4, 6, or 8.

19. The use according to claim 16-18, wherein the cake is an egg and/or fat reduced cake.

20. The use according to claim 16-19, wherein the cake is an emulsifier-free cake.


SEQUENCE LISTING

<110>  DSM IP Assets B.V.

<120>  Method to produce cake

<130>  27946-EP-EPA

<160>  9

<170>  PatentIn version 3.5

<210>  1
<211>  1038
<212>  DNA
<213>  Artificial sequence

<220>
<223>  DNA L01 lipase gene

<400>  1
atgcttctcc tctccctcct ctccattgtc accctcgctg ttgcttctcc tctgtccgtt      60

gaggagtacg ccaaggccct cgaggagcgt gccgtcaccg tctcctcctc cgagctcaac     120

aacttcaagt tctacatcca gcacggtgct gctgcctact gcaactccga gactgctgct     180

ggtgccaacg tcacctgcac tggcaacgcc tgccccgaga ttgaggccaa cggtgtcacc     240

gttgttgcct ccttcactgg taccaagact ggtatcggtg gctacgtctc caccgacaac     300

accaacaagg agatcgtcct ttctttccgt ggcagcatca acatccgcaa ctggctgacc     360

aacctggact tcggccagga tgactgctct ctgacctccg gctgcggtgt ccactccggt     420

ttccagcgtg cctgggagga gattgccgac aacctgaccg ctgctgttgc caaggccaag     480

actgccaacc ccgactacaa ggttgttgcc actggccact ccctgggtgg tgctgttgcc     540

accctggctg gtgccaacct ccgtgctgct ggtacccccc tcgacatcta cacctacggc     600

tctccccgtg tcggcaacgc cgagcttgct gagttcatct ccaaccagac tggtggtgag     660

ttccgtgtca cccacggtga tgaccccgtc ccccgtcttc ctcctctgat cttcggctac     720

cgccacacct cccccgagta ctggctcgat ggcagcggtg gtgacaagat caactacacc     780

atcaacgaca tcaaggtctg cgagggtgct gccaacctgc agtgcaacgg tggtaccctg     840

ggtctcgaca ttgctgctca cctgcactac ttccaggcca ctgatgcctg caacgccggt     900

ggtttcagct ggcgccgcta ccgctctgct gagagcgttg acaagcgtgc caccatgact     960

gatgctgagc tcgagaagaa gctcaacagc tacgtgcaga tggacaagga gtacgtcaag    1020

aacaaccagg ctcgctcc                                                  1038

<210>  2
<211>  346
<212>  PRT
<213>  Artificial sequence

<220>
<223>  L01 lipase protein

<400>  2

Met Leu Leu Leu Ser Leu Leu Ser Ile Val Thr Leu Ala Val Ala Ser
1               5                   10                  15

Pro Leu Ser Val Glu Glu Tyr Ala Lys Ala Leu Glu Glu Arg Ala Val
            20                  25                  30

Thr Val Ser Ser Ser Glu Leu Asn Asn Phe Lys Phe Tyr Ile Gln His
        35                  40                  45

Gly Ala Ala Ala Tyr Cys Asn Ser Glu Thr Ala Ala Gly Ala Asn Val
    50                  55                  60

Thr Cys Thr Gly Asn Ala Cys Pro Glu Ile Glu Ala Asn Gly Val Thr
65                  70                  75                  80

Val Val Ala Ser Phe Thr Gly Thr Lys Thr Gly Ile Gly Gly Tyr Val
                85                  90                  95

Ser Thr Asp Asn Thr Asn Lys Glu Ile Val Leu Ser Phe Arg Gly Ser
            100                 105                 110

Ile Asn Ile Arg Asn Trp Leu Thr Asn Leu Asp Phe Gly Gln Asp Asp
        115                 120                 125

Cys Ser Leu Thr Ser Gly Cys Gly Val His Ser Gly Phe Gln Arg Ala
    130                 135                 140

Trp Glu Glu Ile Ala Asp Asn Leu Thr Ala Ala Val Ala Lys Ala Lys
145                 150                 155                 160

Thr Ala Asn Pro Asp Tyr Lys Val Val Ala Thr Gly His Ser Leu Gly
                165                 170                 175

Gly Ala Val Ala Thr Leu Ala Gly Ala Asn Leu Arg Ala Ala Gly Thr
            180                 185                 190

Pro Leu Asp Ile Tyr Thr Tyr Gly Ser Pro Arg Val Gly Asn Ala Glu
      195                 200                 205

Leu Ala Glu Phe Ile Ser Asn Gln Thr Gly Gly Glu Phe Arg Val Thr
    210                 215                 220

His Gly Asp Asp Pro Val Pro Arg Leu Pro Pro Leu Ile Phe Gly Tyr
225                 230                 235                 240

Arg His Thr Ser Pro Glu Tyr Trp Leu Asp Gly Ser Gly Gly Asp Lys
                245                 250                 255

Ile Asn Tyr Thr Ile Asn Asp Ile Lys Val Cys Glu Gly Ala Ala Asn
            260                 265                 270

Leu Gln Cys Asn Gly Gly Thr Leu Gly Leu Asp Ile Ala Ala His Leu
      275                 280                 285

His Tyr Phe Gln Ala Thr Asp Ala Cys Asn Ala Gly Gly Phe Ser Trp
    290                 295                 300

Arg Arg Tyr Arg Ser Ala Glu Ser Val Asp Lys Arg Ala Thr Met Thr
305                 310                 315                 320

Asp Ala Glu Leu Glu Lys Lys Leu Asn Ser Tyr Val Gln Met Asp Lys
                325                 330                 335

Glu Tyr Val Lys Asn Asn Gln Ala Arg Ser
            340                 345

<210>  3
<211>  1038
<212>  DNA
<213>  Artificial sequence

<220>
<223>  DNA L02 lipase gene

<400>  3
atgcttctcc tctccctcct ctccattgtc accctcgctg ttgcttctcc tctgtccgtt        60

gaggagtacg ccaaggccct cgaggagcgt gccgtcaccg tctcctcctc cgagctcaac       120

aacttcaagt tctacatcca gcacggtgct gctgcctact gcaactccga gactgctgct       180

ggtgccaagg tcacctgctc caacaacggc tgccccgagg ttgaggccaa cggtgtcacc 240

gttgttgcct ccttcgtcgg taccaagacc ggtatcggtg gctacgtggc caccgacaac 300

gcccgcaagg agatcgtcct ctccttccgt ggcagcatca acatccgcaa ctggctgacc 360

aacctggact tcggccagga ggactgctct ctgacctccg gctgcggtgt ccactccggt 420

ttccagcgtg cctgggagga gattgccgac aacttgactg ctgctgttgc caaggccaag 480

actgccaacc ccgactacaa ggtcgtcagc actggccact ctcttggtgg tgctgttgcc 540

accctggctg ctgccaacct ccgtgtcggt ggtactcctc ttgacatcta cacctacggc 600

tctccccgtg tcggcaacgc cgagctctcc gctttcgtct ccaaccagac tggtggtgag 660

ttccgtgtca cccacggtga tgaccccgtc ccccgtcttc ctcctctgat cttcggctac 720

cgccacacct cccccgagta ctggctcgat ggcagcggtg gtgacaaggt cgactacacc 780

atcaacgaca tcaaggtctg cgagggtgct gccaacctgc agtgcaacgg tggtaccctg 840

ggtctcgaca ttgctgctca cctgcactac ttccaggcca ctgatgcctg caacgccggt 900

ggtttcagct ggcgccgcta ccgctctgct gagagcgttg acaagcgtgc caccatgact 960

gatgctgagc tcgagaagaa gctcaacagc tacgtgcaga tggacaagga gtacgtcaag 1020

aacaaccagg ctcgctcc 1038

<210> 4
<211> 346
<212> PRT
<213> Artificial sequence

<220>
<223> L02 lipase protein

<400> 4

Met Leu Leu Leu Ser Leu Leu Ser Ile Val Thr Leu Ala Val Ala Ser
1               5                   10                  15

Pro Leu Ser Val Glu Glu Tyr Ala Lys Ala Leu Glu Glu Arg Ala Val
            20                  25                  30

Thr Val Ser Ser Ser Glu Leu Asn Asn Phe Lys Phe Tyr Ile Gln His
        35                  40                  45

Gly Ala Ala Ala Tyr Cys Asn Ser Glu Thr Ala Ala Gly Ala Lys Val
    50                  55                  60

Thr Cys Ser Asn Asn Gly Cys Pro Glu Val Glu Ala Asn Gly Val Thr
65                  70                  75                  80

Val Val Ala Ser Phe Val Gly Thr Lys Thr Gly Ile Gly Gly Tyr Val
                85                  90                  95

Ala Thr Asp Asn Ala Arg Lys Glu Ile Val Leu Ser Phe Arg Gly Ser
            100                 105                 110

Ile Asn Ile Arg Asn Trp Leu Thr Asn Leu Asp Phe Gly Gln Glu Asp
        115                 120                 125

Cys Ser Leu Thr Ser Gly Cys Gly Val His Ser Gly Phe Gln Arg Ala
    130                 135                 140

Trp Glu Glu Ile Ala Asp Asn Leu Thr Ala Ala Val Ala Lys Ala Lys
145                 150                 155                 160

Thr Ala Asn Pro Asp Tyr Lys Val Val Ser Thr Gly His Ser Leu Gly
                165                 170                 175

Gly Ala Val Ala Thr Leu Ala Ala Ala Asn Leu Arg Val Gly Gly Thr
            180                 185                 190

Pro Leu Asp Ile Tyr Thr Tyr Gly Ser Pro Arg Val Gly Asn Ala Glu
        195                 200                 205

Leu Ser Ala Phe Val Ser Asn Gln Thr Gly Gly Glu Phe Arg Val Thr
    210                 215                 220

His Gly Asp Asp Pro Val Pro Arg Leu Pro Pro Leu Ile Phe Gly Tyr
225                 230                 235                 240

Arg His Thr Ser Pro Glu Tyr Trp Leu Asp Gly Ser Gly Gly Asp Lys
                245                 250                 255

Val Asp Tyr Thr Ile Asn Asp Ile Lys Val Cys Glu Gly Ala Ala Asn
            260                 265                 270

Leu Gln Cys Asn Gly Gly Thr Leu Gly Leu Asp Ile Ala Ala His Leu
        275                 280                 285

His Tyr Phe Gln Ala Thr Asp Ala Cys Asn Ala Gly Gly Phe Ser Trp

290                 295                 300

Arg Arg Tyr Arg Ser Ala Glu Ser Val Asp Lys Arg Ala Thr Met Thr
305                 310                 315                 320

Asp Ala Glu Leu Glu Lys Lys Leu Asn Ser Tyr Val Gln Met Asp Lys
                325                 330                 335

Glu Tyr Val Lys Asn Asn Gln Ala Arg Ser
            340                 345

<210>  5
<211>  1038
<212>  DNA
<213>  Artificial sequence

<220>
<223>  DNA L03 lipase gene

<400>  5
atgcttctcc tctccctcct ctccattgtc accctcgctg ttgcttctcc tctgtccgtt        60

gaggagtacg ccaaggccct cgaggagcgt gccgtcaccg tctcctcctc cgagctcaac       120

aacttcaagt tctacatcca gcacggtgct gctgcctact gcaactccga gactgctgct       180

ggtgccaagg tcacctgctc tggcaacggc tgccccgagg ttgaggccaa cggtgtcacc       240

gttgttgcct ccttcaccgg taccaagacc ggtatcggtg gctacgtggc caccgacaac       300

gcccgcaagg agatcgtcct ctccttccgt ggcagcatca acatccgcaa ctggctgacc       360

aacctggact tcggccagga tgactgctct ctgacctccg gctgcggtgt ccactccggt       420

ttccagcgtg cctgggagga gattgccgac aacttgactg ctgctgttgc caaggccaag       480

actgccaacc ccgactacaa ggtcgtcgcc actggccact ccctgggtgg tgctgttgcc       540

accctggctg gtgccaacct ccgtgtcggt ggtactcctc ttgacatcta cacctacggc       600

tctccccgtg tcggcaacgc cgagcttgct gctttcgtct ccaaccagac tggtggtgag       660

ttccgtgtca cccacggtga tgaccccgtc ccccgtcttc ctcctctgat cttcggctac        720

cgccacacct cccccgagta ctggctcgat ggcagcggtg gtgacaagat cgactacacc       780

atcaacgaca tcaaggtctg cgagggtgct gccaacctgc agtgcaacgg tggtaccctg       840

ggtctcgaca ttgctgctca cctgcactac ttccaggcca ctgatgcctg caacgccggt       900

ggtttcagct ggcgccgcta ccgctctgct gagagcgttg acaagcgtgc caccatgact       960

gatgctgagc tcgagaagaa gctcaacagc tacgtgcaga tggacaagga gtacgtcaag      1020

aacaaccagg ctcgctcc                                                    1038

<210>  6
<211>  346
<212>  PRT
<213>  Artificial sequence

<220>
<223>  L03 lipase protein

<400>  6

Met Leu Leu Leu Ser Leu Leu Ser Ile Val Thr Leu Ala Val Ala Ser
1               5                   10                  15

Pro Leu Ser Val Glu Glu Tyr Ala Lys Ala Leu Glu Glu Arg Ala Val
            20                  25                  30

Thr Val Ser Ser Ser Glu Leu Asn Asn Phe Lys Phe Tyr Ile Gln His
        35                  40                  45

Gly Ala Ala Ala Tyr Cys Asn Ser Glu Thr Ala Ala Gly Ala Lys Val
    50                  55                  60

Thr Cys Ser Gly Asn Gly Cys Pro Glu Val Glu Ala Asn Gly Val Thr
65                  70                  75                  80

Val Val Ala Ser Phe Thr Gly Thr Lys Thr Gly Ile Gly Gly Tyr Val
                85                  90                  95

Ala Thr Asp Asn Ala Arg Lys Glu Ile Val Leu Ser Phe Arg Gly Ser
            100                 105                 110

Ile Asn Ile Arg Asn Trp Leu Thr Asn Leu Asp Phe Gly Gln Asp Asp
        115                 120                 125

Cys Ser Leu Thr Ser Gly Cys Gly Val His Ser Gly Phe Gln Arg Ala
    130                 135                 140

Trp Glu Glu Ile Ala Asp Asn Leu Thr Ala Ala Val Ala Lys Ala Lys
145                 150                 155                 160

Thr Ala Asn Pro Asp Tyr Lys Val Val Ala Thr Gly His Ser Leu Gly
                165                 170                 175

Gly Ala Val Ala Thr Leu Ala Gly Ala Asn Leu Arg Val Gly Gly Thr
            180                 185                 190

Pro Leu Asp Ile Tyr Thr Tyr Gly Ser Pro Arg Val Gly Asn Ala Glu
        195                 200                 205

Leu Ala Ala Phe Val Ser Asn Gln Thr Gly Gly Glu Phe Arg Val Thr
    210                 215                 220

His Gly Asp Asp Pro Val Pro Arg Leu Pro Pro Leu Ile Phe Gly Tyr
225                 230                 235                 240

Arg His Thr Ser Pro Glu Tyr Trp Leu Asp Gly Ser Gly Gly Asp Lys
                245                 250                 255

Ile Asp Tyr Thr Ile Asn Asp Ile Lys Val Cys Glu Gly Ala Ala Asn
            260                 265                 270

Leu Gln Cys Asn Gly Gly Thr Leu Gly Leu Asp Ile Ala Ala His Leu
        275                 280                 285

His Tyr Phe Gln Ala Thr Asp Ala Cys Asn Ala Gly Gly Phe Ser Trp
    290                 295                 300

Arg Arg Tyr Arg Ser Ala Glu Ser Val Asp Lys Arg Ala Thr Met Thr
305                 310                 315                 320

Asp Ala Glu Leu Glu Lys Lys Leu Asn Ser Tyr Val Gln Met Asp Lys
                325                 330                 335

Glu Tyr Val Lys Asn Asn Gln Ala Arg Ser
            340                 345

<210>  7
<211>  1038
<212>  DNA
<213>  Artificial sequence

<220>
<223>  DNA L04 lipase gene

<400>  7
atgcttctcc tctccctcct ctccattgtc accctcgctg ttgcttctcc tctgtccgtt       60

gaggagtacg ccaaggccct cgaggagcgt gccgtcaccg tctcctcctc cgagctcaac      120

aacttcaagt tctacatcca gcacggtgct gctgcctact gcaactccga gactgctgct 180

ggtgccaacg tcacctgctc tggcaacggc tgccccgagg ttgaggccaa cggtgtcacc 240

gttgttgcct ccttcaccgg taccaagacc ggtatcggtg gctacgtcgc caccgacaac 300

gcccgcaagg agatcgtcct ctccttccgt ggcagcatca acatccgcaa ctggctgacc 360

aacctggact tcggccagga tgactgctct ctgacctccg gctgcggtgt ccactccggt 420

ttccagcgtg cctgggagga gattgccgac aacttgactg ctgctgttgc caaggccaag 480

actgccaacc ccgactacaa ggttgttgcc actggccact ctcttggtgg tgctgttgcc 540

actctggctg gtgccaacct ccgtgtcggt ggtacccccc tcgacatcta cacctacggc 600

tctcctcgtg tcggcaacgc cgagcttgct gctttcgtct ccaaccaggc tggtggtgag 660

ttccgtgtca cccacggtga tgaccccgtc ccccgtcttc ctcctctgat cttcggctac 720

cgccacacct cccccgagta ctggctcgat ggcagcggtg gtgacaagat cgactacacc 780

atcaacgaca tcaaggtctg cgagggtgct gccaacctgc agtgcaacgg tggtaccctg 840

ggtctcgaca ttgctgctca cctgcactac ttccaggcca ctgatgcctg caacgccggt 900

ggtttcagct ggcgccgcta ccgctctgct gagagcgttg acaagcgtgc caccatgact 960

gatgctgagc tcgagaagaa gctcaacagc tacgtgcaga tggacaagga gtacgtcaag 1020

aacaaccagg ctcgctcc 1038

<210> 8
<211> 346
<212> PRT
<213> Artificial sequence

<220>
<223> L04 lipase protein

<400> 8

Met Leu Leu Leu Ser Leu Leu Ser Ile Val Thr Leu Ala Val Ala Ser
1               5                   10                  15

Pro Leu Ser Val Glu Glu Tyr Ala Lys Ala Leu Glu Glu Arg Ala Val
            20                  25                  30

Thr Val Ser Ser Ser Glu Leu Asn Asn Phe Lys Phe Tyr Ile Gln His
        35                  40                  45

Gly Ala Ala Ala Tyr Cys Asn Ser Glu Thr Ala Ala Gly Ala Asn Val

50                  55                  60

Thr Cys Ser Gly Asn Gly Cys Pro Glu Val Glu Ala Asn Gly Val Thr
65                  70                  75                  80

Val Val Ala Ser Phe Thr Gly Thr Lys Thr Gly Ile Gly Gly Tyr Val
                85                  90                  95

Ala Thr Asp Asn Ala Arg Lys Glu Ile Val Leu Ser Phe Arg Gly Ser
            100                 105                 110

Ile Asn Ile Arg Asn Trp Leu Thr Asn Leu Asp Phe Gly Gln Asp Asp
        115                 120                 125

Cys Ser Leu Thr Ser Gly Cys Gly Val His Ser Gly Phe Gln Arg Ala
    130                 135                 140

Trp Glu Glu Ile Ala Asp Asn Leu Thr Ala Ala Val Ala Lys Ala Lys
145                 150                 155                 160

Thr Ala Asn Pro Asp Tyr Lys Val Val Ala Thr Gly His Ser Leu Gly
                165                 170                 175

Gly Ala Val Ala Thr Leu Ala Gly Ala Asn Leu Arg Val Gly Gly Thr
            180                 185                 190

Pro Leu Asp Ile Tyr Thr Tyr Gly Ser Pro Arg Val Gly Asn Ala Glu
        195                 200                 205

Leu Ala Ala Phe Val Ser Asn Gln Ala Gly Gly Glu Phe Arg Val Thr
    210                 215                 220

His Gly Asp Asp Pro Val Pro Arg Leu Pro Pro Leu Ile Phe Gly Tyr
225                 230                 235                 240

Arg His Thr Ser Pro Glu Tyr Trp Leu Asp Gly Ser Gly Gly Asp Lys
                245                 250                 255

Ile Asp Tyr Thr Ile Asn Asp Ile Lys Val Cys Glu Gly Ala Ala Asn
            260                 265                 270

Leu Gln Cys Asn Gly Gly Thr Leu Gly Leu Asp Ile Ala Ala His Leu
        275                 280                 285

His Tyr Phe Gln Ala Thr Asp Ala Cys Asn Ala Gly Gly Phe Ser Trp
    290                 295                 300

Arg Arg Tyr Arg Ser Ala Glu Ser Val Asp Lys Arg Ala Thr Met Thr
305                 310                 315                 320

Asp Ala Glu Leu Glu Lys Lys Leu Asn Ser Tyr Val Gln Met Asp Lys
                325                 330                 335

Glu Tyr Val Lys Asn Asn Gln Ala Arg Ser
            340                 345

<210>  9
<211>  686
<212>  PRT
<213>  Bacillus stearothermophilus

<400>  9

Ser Ser Ser Ala Ser Val Lys Gly Asp Val Ile Tyr Gln Ile Ile Ile
1               5                   10                  15

Asp Arg Phe Tyr Asp Gly Asp Thr Thr Asn Asn Asn Pro Ala Lys Ser
            20                  25                  30

Tyr Gly Leu Tyr Asp Pro Thr Lys Ser Lys Trp Lys Met Tyr Trp Gly
        35                  40                  45

Gly Asp Leu Glu Gly Val Arg Gln Lys Leu Pro Tyr Leu Lys Gln Leu
    50                  55                  60

Gly Val Thr Thr Ile Trp Leu Ser Pro Val Leu Asp Asn Leu Asp Thr
65                  70                  75                  80

Leu Ala Gly Thr Asp Asn Thr Gly Tyr His Gly Tyr Trp Thr Arg Asp
                85                  90                  95

Phe Lys Gln Ile Glu Glu His Phe Gly Asn Trp Thr Thr Phe Asp Thr
            100                 105                 110

Leu Val Asn Asp Ala His Gln Asn Gly Ile Lys Val Ile Val Asp Phe
        115                 120                 125

Val Pro Asn His Ser Thr Pro Phe Lys Ala Asn Asp Ser Thr Phe Ala
    130                 135                 140

Glu Gly Gly Ala Leu Tyr Asn Asn Gly Thr Tyr Met Gly Asn Tyr Phe
145                 150                 155                 160

Asp Asp Ala Thr Lys Gly Tyr Phe His His Asn Gly Asp Ile Ser Asn
                165                 170                 175

Trp Asp Asp Arg Tyr Glu Ala Gln Trp Lys Asn Phe Thr Asp Pro Ala
            180                 185                 190

Gly Phe Ser Leu Ala Asp Leu Ser Gln Glu Asn Gly Thr Ile Ala Gln
        195                 200                 205

Tyr Leu Thr Asp Ala Ala Val Gln Leu Val Ala His Gly Ala Asp Gly
    210                 215                 220

Leu Arg Ile Asp Ala Val Lys His Phe Asn Ser Gly Phe Ser Lys Ser
225                 230                 235                 240

Leu Ala Asp Lys Leu Tyr Gln Lys Lys Asp Ile Phe Leu Val Gly Glu
                245                 250                 255

Trp Tyr Gly Asp Asp Pro Gly Thr Ala Asn His Leu Glu Lys Val Arg
            260                 265                 270

Tyr Ala Asn Asn Ser Gly Val Asn Val Leu Asp Phe Asp Leu Asn Thr
        275                 280                 285

Val Ile Arg Asn Val Phe Gly Thr Phe Thr Gln Thr Met Tyr Asp Leu
    290                 295                 300

Asn Asn Met Val Asn Gln Thr Gly Asn Glu Tyr Lys Tyr Lys Glu Asn
305                 310                 315                 320

Leu Ile Thr Phe Ile Asp Asn His Asp Met Ser Arg Phe Leu Ser Val
                325                 330                 335

Asn Ser Asn Lys Ala Asn Leu His Gln Ala Leu Ala Phe Ile Leu Thr
            340                 345                 350

Ser Arg Gly Thr Pro Ser Ile Tyr Tyr Gly Thr Glu Gln Tyr Met Ala

355                 360                 365

Gly Gly Asn Asp Pro Tyr Asn Arg Gly Met Met Pro Ala Phe Asp Thr
    370                 375                 380

Thr Thr Thr Ala Phe Lys Glu Val Ser Thr Leu Ala Gly Leu Arg Arg
385                 390                 395                 400

Asn Asn Ala Ala Ile Gln Tyr Gly Thr Thr Thr Gln Arg Trp Ile Asn
                405                 410                 415

Asn Asp Val Tyr Ile Tyr Glu Arg Lys Phe Phe Asn Asp Val Val Leu
            420                 425                 430

Val Ala Ile Asn Arg Asn Thr Gln Ser Ser Tyr Ser Ile Ser Gly Leu
        435                 440                 445

Gln Thr Ala Leu Pro Asn Gly Ser Tyr Ala Asp Tyr Leu Ser Gly Leu
    450                 455                 460

Leu Gly Gly Asn Gly Ile Ser Val Ser Asn Gly Ser Val Ala Ser Phe
465                 470                 475                 480

Thr Leu Ala Pro Gly Ala Val Ser Val Trp Gln Tyr Ser Thr Ser Ala
                485                 490                 495

Ser Ala Pro Gln Ile Gly Ser Val Ala Pro Asn Met Gly Ile Pro Gly
            500                 505                 510

Asn Val Val Thr Ile Asp Gly Lys Gly Phe Gly Thr Thr Gln Gly Thr
        515                 520                 525

Val Thr Phe Gly Gly Val Thr Ala Thr Val Lys Ser Trp Thr Ser Asn
    530                 535                 540

Arg Ile Glu Val Tyr Val Pro Asn Met Ala Ala Gly Leu Thr Asp Val
545                 550                 555                 560

Lys Val Thr Ala Gly Gly Val Ser Ser Asn Leu Tyr Ser Tyr Asn Ile
                565                 570                 575

Leu Ser Gly Thr Gln Thr Ser Val Val Phe Thr Val Lys Ser Ala Pro
            580                 585                 590

Pro Thr Asn Leu Gly Asp Lys Ile Tyr Leu Thr Gly Asn Ile Pro Glu
        595                 600                 605

Leu Gly Asn Trp Ser Thr Asp Thr Ser Gly Ala Val Asn Asn Ala Gln
    610                 615                 620

Gly Pro Leu Leu Ala Pro Asn Tyr Pro Asp Trp Phe Tyr Val Phe Ser
625                 630                 635                 640

Val Pro Ala Gly Lys Thr Ile Gln Phe Lys Phe Phe Ile Lys Arg Ala
                645                 650                 655

Asp Gly Thr Ile Gln Trp Glu Asn Gly Ser Asn His Val Ala Thr Thr
            660                 665                 670

Pro Thr Gly Ala Thr Gly Asn Ile Thr Val Thr Trp Gln Asn
        675                 680                 685